# FASCICULE DE BREVET EUROPEEN

(11) **EP 0 570 489 B1**
(45) Date de publication et mention de la délivrance du brevet: **06.05.1999**
(21) Numéro de dépôt: 92905897.2
(22) Date de dépôt: 05.02.1992
(51) Int. Cl.: C07K 14/00, A61K 39/015, C12N 15/30, C12P 21/08, G01N 33/569

(54) **SEQUENCES PEPTIDIQUES SPECIFIQUES DES STADES HEPATIQUES DE $i(P. FALCIPARUM) PORTEUSES D'EPITOPES CAPABLES DE STIMULER LES LYMPHOCYTES T.**
PEPTIDSEQUENZEN, SPEZIFISCH FÜR DIE HEPATITISCHE PHASE VON P. FALCIPARUM, EPITOPE TRAGEND; DIE DIE FÄHIGKEIT BESITZEN, DIE T-LYMPHOZYTEN ANZUREGEN
LIVER-STAGE-SPECIFIC PEPTIDE SEQUENCES OF $i(P. FALCIPARUM) BEARING EPITOPES CAPABLE OF STIMULATING THE T LYMPHOCYTES

(30) Priorité: 05.02.1991 FR 9101286
(43) Date de publication de la demande: 24.11.1993
(73) Titulaire: INSTITUT PASTEUR, 75724 Paris Cédex 15 (FR)
(72) Inventeur: GUERIN-MARCHAND, Claudine, F-75012 Paris (FR); DRUILHE, Pierre, F-75016 Paris (FR)
(74) Mandataire: Gutmann, Ernest
(86) Numéro de dépôt international: FR9200104
(87) Numéro de publication internationale: WO9213884

(56) Documents cités:
- EP-A- 407 230
- WO-A-88/05785
- WO-A-90/06130
- WO-A-92/05193
- JOURNAL OF IMMUNOLOGY, vol. 145, no. 5, 01 September 1990, Baltimore, MD (US); A. LONDONO et al., pp. 1557-1563
- NATURE, vol. 329, no. 6135, 16 September 1987, London (GB); G. GUERIN-MARCHAND et al., pp. 264-267
- BULLETIN OF THE WHO, vol. 68, no. SUP, 1990, GENEVA (CH); C. MARCHAND et al., pp. 158-164
- CHEMICAL ABSTRACTS, vol. 113, no. 21, 19 November 1990, Columbus, OH (US); M.R. HOLLINGDALE et al., p. 534, AN 189314w

## Description

Les parasites responsables du paludisme chez l'homme, dont notamment Plasmodium falciparum ou Plasmodium vivax pour ne citer que les principaux d'entre eux, présentent chez l'hôte humain des morphologies différentes et expriment des antigènes différents en fonction de leur localisation dans l'organisme de l'hôte infecté. Les différences morphologiques et antigéniques de ces parasites au cours de leurs cycles de vie chez l'homme, permettent de définir au moins quatre stades de développement distincts.

Le tout premier stade de développement du parasite chez l'homme correspond à la forme sporozoïte introduite dans le sang de l'hôte, par piqûres d'insectes porteurs du parasite. Le second stade correspond au passage du parasite dans le foie et à l'infection des cellules hépatiques dans lesquelles les parasites se développent pour former les schizontes hépatiques qui libèrent par éclatement les mérozoïtes hépatiques. Le troisième stade est caractérisé par l'infection des érythrocytes sanguins par les formes asexuées (mérozoïtes) du parasite; ce stade érythrocytaire de développement du parasite correspond à la phase pathogène de la maladie.

Le quatrième stade correspond à la formation des formes sexuées (ou gamétocytes) qui deviendront les gamètes extra-cellulaires chez le moustique.

On sait que de nombreuses études ont été entreprises pour isoler à partir des souches de parasites infectantes pour un hôte humain des fractions polypeptidiques, d'une part pour assurer le diagnostic in vitro du paludisme par détection des anticorps correspondants, et, d'autre part, pour tenter de vacciner contre le paludisme.

Par exemple, des banques de cADNs (ADN complémentaires) clones dérivés des sporozoïtes de Plasmodium falciparum ont été établies par ENEA et coll (1984) Science, vol. 225, 628-630. Il a été reconnu que ces banques comportaient des clones susceptibles d'exprimer des polypeptides immunogéniques contenant des unités répétitives de 4 acides aminés spécifiques de l'antigène circumsporozoïtaire (de P. falciparum).

Toutefois, peu de travaux ont été effectués sur les formes hépatiques des parasites responsables du paludisme. La morphologie des formes hépatiques a été décrite pour la première fois en 1948 à partir de biopsies de volontaires humains infectés (Trans. Roy. Soc. Trop. Med. Hyg., 41, 785 (1948)). Un antigène spécifique du stade hépatique de P. falciparum a pu être décrit dans le foie de singes d'Amérique du Sud insensibles aux formes sanguines du parasite, mais chez lesquels les formes hépatiques peuvent se développer (Am. J. Trop. Med. Hyg., 33, (3) 336-341 (1984)).

La détection de la localisation des antigènes spécifiques du parasite lorsqu'il est au stade hépatique (ci-après désigné par LS antigenés pour "Liver Stage" antigènes) a été réalisée par immunofluorescence tout au long des étapes de maturation du schizonte. Il est localisé à la périphérie du parasite de taille 5 à 40 microns ; par la suite ils sont distribués entre les cytomères ou paquets de mérozoïtes, lorsque les schizontes atteignent entre 50 et 100 microns. Ils se distinguent des antigènes de surface des sporozoites et des antigènes partagés par les schizontes du sang et du foie qui donnent une image d'immunofluorescence interne au parasite.

Bien qu'il soit désormais possible de cultiver des formes hépatiques de P. falciparum dans des hépatocytes humains (Science, 227, 440 (1985)), le faible taux d'obtention de formes matures du parasite par les méthodes de culture in vitro et in vivo ne permet pas l'analyse biochimique de l'antigène produit au stade hépatique.

La demande de brevet international WO 90/06130 décrit une protéine antigènique isolée de sporozoite de Plasmodium falciparum.

Il a également été observé que les individus atteints de paludisme possèdent un taux d'anticorps dirigés contre les LSA très élevés. Les LS antigènes semblent être des immunogènes très puissant, parmi les plus puissants de tous les antigènes synthétisés aux différents stades de développement du parasite.

Un des buts de la présente invention est précisément de disposer de nouvelles compositions pour la vaccination chez l'homme contre le paludisme provoqué par P. falciparum.

L'invention a également pour objet le diagnostic in vitro de l'infection d'un individu par P. falciparum dans des conditions plus sensibles que ne le permettent les méthodes actuelles.

Une molécule exprimée spécifiquement au cours de la phase hépatique a été identifiée par criblage avec des serums polyclonaux d'une banque d'ADN génomique clonée dans un vecteur d'expression (GUERIN-MARCHAND, C. et al ; Nature, 329, 164-167, (1987)). Cette molécule représente une partie d'un antigène appelé LSA (Liver Stage Specific antigen), et est constituée de motifs répétitifs de 17 acides aminés et semble très immunogène dans les conditions naturelles d'exposition à la maladie.

Ces motifs répétitifs de 17 acides aminés sont représentés par la formule : 
Leu-Ala-Lys-Glu-Lys-Leu-Gln-X-Gln-Gln-Ser-Asp-Leu-
Glu-Gln-Glu-Arg
 dans laquelle X est Glu ou Gly.

L'invention a pour objet des séquences peptidiques spécifiques des stades hépatiques de P. falciparum porteuses d'épitopes capables de stimuler les lymphocytes T (en particulier les lymphocytes T cytotoxiques).

L'invention concerne plus particulièrement des molécules, ou compositions peptidiques ou polypeptidiques, caractérisées par la présence dans leur structure d'une ou de plusieurs séquences peptidiques porteuses de tout ou partie d'un ou plusieurs épitope(s) T (épitopes impliqués dans la stimulation des lymphocytes T), et le cas échéant d'autres épitopes, notamment des épitopes B (épitopes correspondant aux anticorps produits par des lymphocytes B), caractéristiques des protéines résultant de l'activité infectieuse de P. falciparum dans les cellules hépatiques.

Il a été également montré que cette protéine LSA est capable de stimuler des lymphocytes T (Chemical Abtracts, vol; 113, n0 21, 1990, abstracts n° 189314W). De même, il a été décrit, dans la demande de brevet international WO 88/05785 (Institut Pasteur), une molécule comportant une séquence peptidique porteuse d'un épitope B caractéristique d'une protéine résultant de l'activité infectieuse de P. falciparum dans les cellules hépatiques. De même, il a été décrit dans la demande de brevet européen 0 407 230 (Institut Pasteur) un polypeptide comportant un séquence peptidique porteuse d'un épitope spécifique d'une protéine produite au stade sporozoite et dans les hépatocytes infecté par P. falciparum.

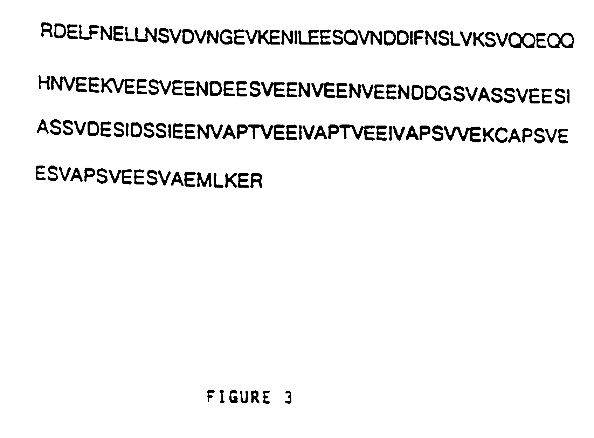

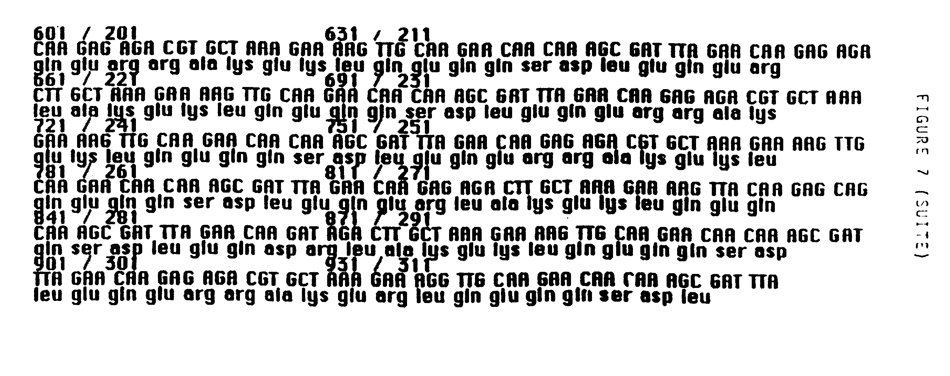

Ainsi, la présente invention concerne une molécule, ou un polypeptide, comportant au moins une séquence peptidique qui comprend au moins 4 à 5 acides aminés et qui est porteuse de tout ou partie d'un ou plusieurs épitopes caractéristiques d'une protéine produite dans les hépatocytes infectés par P. falciparum, caractérisée en ce que cette séquence peptidique comprend :
a) soit tout ou partie de l'enchaînement des 153 premiers acides aminés représentés sur la figure 7, et correspondant à la partie 5' du gène LSA ;
b) soit tout ou partie de l'enchaînement d'acides aminés suivant : 
RDELFNELLNSVDVNGEVKENILEESQVNDDIFNSLVKSVQQEQQHNVEEKVE
ESVEENDEESVEENVEENVEENDDGSVASSVEESIASSVDESIDSSIEENVAP
TVEEIVAPTVEEIVAPSVVEKCAPSVEESVAPSVEESVAEMLKER
 représenté sur la figure 3 et désigné ci-après par le polypeptide 729S ;
c) soit tout ou partie des 279 derniers acides aminés représentés sur la figure 10 et correspondant à la partie 3' du gène LSA ;
d) soit tout ou partie de l'enchaînement d'acides aminés suivant : 
RKADTKKNLERKKEHGDILAEDLYGRLEIP
AIELPSENERGYYIPHQSSLPQDNRGNSRD
SKEISIIEKTNRESITTNVEGRRDIHKGHL
EEKKDGSIKPEQKEDKS
 représenté sur la figure 10.

Il sera fait référence dans ce qui suit aux dessins dans lesquels:
- la figure 1 représente une protéine recombinante de 316 acides aminés de l'invention, désignée ci-après par antigène 536 ou protéine LSA-R-NR,
- la figure 2 fournit la séquence nucléotidique d'un des acides nucléiques recombinants étudiés (clone DG536) et codant pour le polypeptide LSA-R-NR,
- la figure 3 représente un polypeptide de 151 acides aminés de l'invention, désigné ci-après par antigène 729S,
- la figure 4 correspond à la séquence nucléotidique du clone DG729S codant pour le polypeptide de la figure 3 (adaptateurs Eco RI en gras),
- la figure 5 représente les séquences polypeptidiques des antigènes LSA-TER, 729S-NRI, 729S-NRII, 729S-Rep,
- la figure 6 représente la séquence nucléotidique du gène LSA, dans sa partie 5',
- la figure 7 représente la séquence codante de la partie 5' du gène LSA et la séquence polypeptidique correspondante,
- la figure 8 décrit la partie 3' du gène LSA,
- la figure 9 donne la séquence de la partie 3' du gène LSA, ainsi que la séquence polypeptidique correspondante,
- la figure 10 reprend les séquences données à la figure 9, jusqu'au codon stop de terminaison et à l'acide aminé terminal.

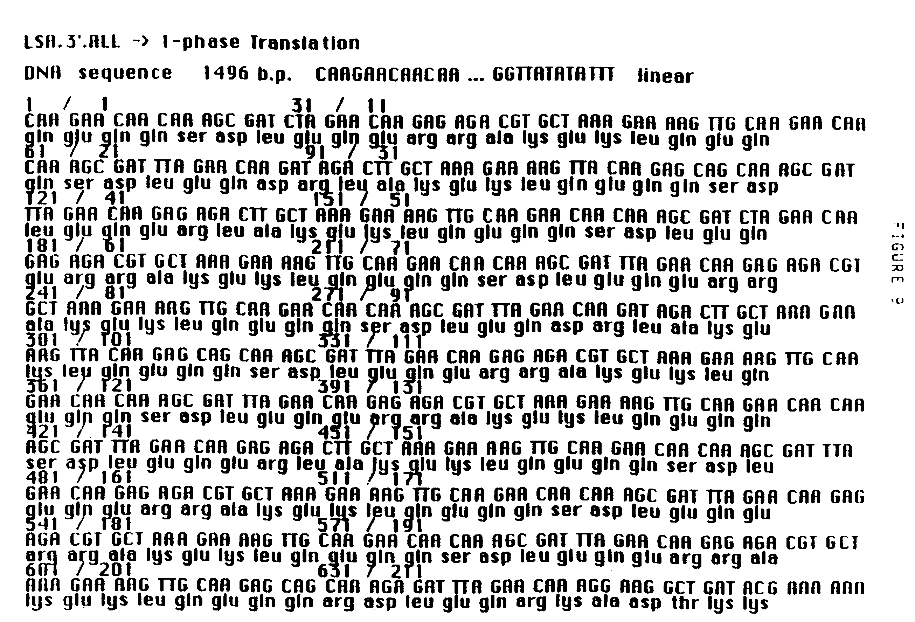

Ainsi la présente invention concerne toute molécule, ou composition polypeptidique, comportant au moins une séquence peptidique porteuse de tout ou partie d'un, ou plusieurs, épitopes caractéristiques d'une protéine produite dans les hépatocytes infectés par P. falciparum, et plus particulièrement porteuse de tout ou partie d'un ou plusieurs épitope(s) T des protéines produites au stade hépatique de P. falciparum, caractérisée en ce que cette séquence peptidique est représentée par tout ou partie de l'enchaînement d'acides aminés représenté sur la figure 9 ou la figure 10, et correspondant à la partie 3' du gène LSA.

L'invention a plus particulièrement pour objet toute molécule, ou composition polypeptidique, comportant au moins une séquence peptidique porteuse de tout ou partie d'un, ou plusieurs, épitopes caractéristiques d'une protéine produite dans les hépatocytes infectés par P. falciparum, et plus particulièrement porteuse de tout ou partie d'un ou plusieurs épitope(s) T des protéines produites au stade hépatique de P. falciparum, caractérisée en ce que cette séquence peptidique est représentée par tout ou partie de l'enchaînement des 279 derniers acides aminés représentés sur la figure 10, cet enchaînement d'acides aminés étant, le cas échéant, précédé par toute ou partie d'un ou plusieurs des enchaînements de 17 acides aminés de formule : 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 dans laquelle :
. X₁ est "Ser" ou "Arg",
. X₂ est "Glu" ou "Asp",
. X₃ est "Arg" ou "Leu",
. X₄ est "Glu" ou "Gly".

A ce titre l'invention concerne plus particulièrement toute molécule, ou composition polypeptidique, comportant au moins une séquence peptidique porteuse de tout ou partie d'un, ou plusieurs, épitopes caractéristiques d'une protéine produite dans les hépatocytes infectés par P. falciparum, et plus particulièrement porteuse de tout ou partie d'un ou plusieurs épitope(s) T des protéines produites au stade hépatique de P. falciparum, caractérisée en ce que cette séquence peptidique est représentée par tout ou partie de l'enchaînement d'acides aminés suivant : 
RKADTKKNLERKKEHGDILAEDLYGRLEIPAIELPS
ENERGYYIPHQSSLPQDNRGNSRDSKEISIIEKTNR
ESITTNVEGRRDIHKGHLEEKKDGSIKPEQKEDKS
 cet enchaînement d'acides aminés étant, le cas échéant, précédé par toute ou partie d'un ou plusieurs des enchaînements de 17 acides aminés de formule : 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
 
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 dans laquelle :
. X₁ est "Ser" ou "Arg",
. X₂ est "Glu" ou "Asp",
. X₃ est "Arg" ou "Leu",
. X₄ est "Glu" ou "Gly".

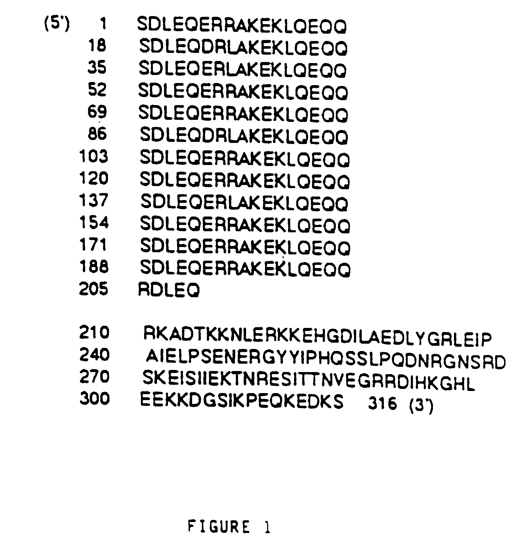

Ainsi la présente invention concerne notamment la séquence peptidique représentée sur la figure 1. Cette séquence est constituée de 316 acides aminés. A l'extrémité 5' se trouvent 209 acides aminés organisés en répétitions de 17 acides aminés répondant aux formules indiquées ci-dessus. Du côté 3', on trouve une partie répétée de 107 acides aminés.

L'invention concerne plus particulièrement tout polypeptide caractérisé par tout ou partie de l'enchaînement d'acides aminés suivant :

LQEQQRDLEQRKADTKKNLERKKEHGDILAEDLYGRLEIPAIELPSENERGYY
IPHQSSLPQDNRGNSRDSKEISIIEKTNRESITTNVEGRRDIHKGHLEEKKDG
SIKPEQKEDKS

Un polypeptide préféré de l'invention est représenté par tout ou partie de l'enchaînement d'acides aminés suivant : DTKKNLERKKEHGDILAEDLYGRLEIP (ce polypeptide étant désigné ci-après par l'expression LSA-NR (LSA-non répété), ou encore par toute séquence issue de l'enchaînement précédent et modifiée par substitution de 40 % maximum des acides aminés et conservant son activité physiologique telle que l'induction d'une réponse des lymphocytes T en particulier des lymphocytes T cytotoxiques.

Une autre molécule polypeptidique particulièrement préférée de l'invention est caractérisée par tout ou partie de l'enchaînement d'acides aminés suivant : ERRAKEKLQEQQRDLEQRKADTKK (ce polypeptide étant désigné ci-après par l'expression LSA-J, ou LSA-jonction, car il est situé à cheval entre la partie répétée et la partie non répétée de la molécule représentée sur la figure 1).

Un autre peptide préféré et désigné LSA-TER, est le suivant :

NSRDSKEISIIEKTNRESITTNVEGRRDIHK

Ces trois derniers polypeptides sont plus particulièrement avantageux en raison de leur amphipaticité les caractérisant, ainsi que de leur conformation tridimensionnelle selon des prédictions réalisées par la technique de Chou et Fassmann.

L'invention a également pour objet toute molécule, ou composition polypeptidique, comportant au moins une séquence peptidique porteuse de tout ou partie d'un ou plusieurs épitopes caractéristiques d'une protéine produite au niveau des stades sporozoïte, hépatique et sanguin (erythrocytaire) de P. falciparum, et plus particulièrement porteuse d'un ou plusieurs épitopes T, caractérisée en ce que cette séquence peptidique est représentée par tout ou partie de l'enchaînement d'acides aminés suivant : 
RDELFNELLNSVDVNGEVKENILEESQVNDDIFNSLVKSVQQEQQHNVEEKVE
ESVEENDEESVEENVEENVEENDDGSVASSVEESIASSVDESIDSSIEENVAP
TVEEIVAPTVEEIVAPSVVEKCAPSVEESVAPSVEESVAEMLKER
 représenté sur la figure 3 et désigné ci-après par le polypeptide 729S.

L'invention a plus particulièrement pour objet la séquence en acides aminés issue de la séquence précédente et caractérisée par tout ou partie de l'enchaînement d'acides aminés suivant :

RDELFNELLNSVDVNGEVKENILEESQVNDDIFNSLVKSVQQEQQHN

Selon un autre mode de réalisation avantageux de l'invention, des séquences intéressantes, issues de l'enchaînement d'acides aminés du polypeptide 729S, sont les suivantes :

- DELFNELLNSVDVNGEVKENILEESQ,
- LEESQVNDDIFSNSLVKSVQQEQQHNV,
- VEKCAPSVEESVAPSVEESVAEMLKER.

Ces séquences sont respectivement désignées 729S-NRI, 729S-NRII, 729S-Rep.

L'invention a également pour objet toute molécule, ou composition polypeptidique, comportant au moins une séquence peptidique porteuse de tout ou partie d'un, ou plusieurs, épitopes caractéristiques d'une protéine produite dans les hépatocytes infectés par P. falciparum, et plus particulièrement porteuse de tout ou partie d'un ou plusieurs épitope(s) T des protéines produites au stade hépatique de P. falciparum, caractérisée en ce que cette séquence peptidique est représentée par tout ou partie de l'enchaînement d'acides aminés représentés sur la figure 7, et correspondant à la partie 5' de gène LSA.

A ce titre l'invention a plus particulièrement pour objet toute molécule, ou composition polypeptidique, comportant au moins une séquence peptidique porteuse de tout ou partie d'un, ou plusieurs, épitopes caractéristiques d'une protéine produite dans les hépatocytes infectés par P. falciparum, et plus particulièrement porteuse de tout ou partie d'un ou plusieurs épitope(s) T des protéines produites au stade hépatique de P. falciparum, caractérisée en ce que cette séquence peptidique est représentée par tout ou partie de l'enchaînement des 153 premiers acides aminés représentés sur la figure 7, cet enchaînement d'acides aminés étant, le cas échéant, suivi par toute ou partie d'un ou plusieurs des enchaînements de 17 acides aminés de formule : 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 dans laquelle :
. X₁ est "Ser" ou "Arg",
. X₂ est "Glu" ou "Asp",
. X₃ est "Arg" ou "Leu",
. X₄ est "Glu" ou "Gly".

L'invention vise également toute molécule, ou composition polypeptidique, comportant au moins une séquence peptidique porteuse de tout ou partie d'un, ou plusieurs, épitopes caractéristiques d'une protéine produite dans les hépatocytes infectés par P. falciparum, et plus particulièrement porteuse de tout ou partie d'un ou plusieurs épitope(s) T des protéines produites au stade hépatique de P. falciparum, caractérisée en ce que cette séquence peptidique comprend successivement :
- tout ou partie de l'enchaînement des 153 premiers acides aminés représentés sur la figure 7,
- le cas échéant tout ou partie d'un ou plusieurs des enchaînements de 17 acides aminés de formule : 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 dans laquelle :
   . X₁ est "Ser" ou "Arg",
   . X₂ est "Glu" ou "Asp",
   . X₃ est "Arg" ou "Leu",
   . X₄ est "Glu" ou "Gly".

- et tout ou partie des 279 derniers acides aminés représentés sur la figure 10.

L'invention concerne également toute composition polypeptidique constituée de plusieurs séquences peptidiques différentes porteuses de tout ou partie d'un, ou plusieurs épitopes caractéristiques d'une protéine produite dans les hépatocytes infectés par P. falciparum, telles que décrites ci-dessus.

D'une manière générale, par toute ou partie d'une séquence peptidique de l'invention, on entend tout enchaînement comprenant au moins 4 à 5 acides aminés jusqu'au nombre maximal d'acides aminés des séquences décrites ci-dessus.

Il va de soi que les fonctions réactives libres que sont susceptibles de posséder certains acides aminés entrant dans la constitution des molécules selon l'invention, notamment les groupes carboxyles libres portés par les groupes Glu ou par l'acide aminé C-terminal, d'une part, et/ou les groupes libres portés par l'acide aminé N-terminal ou par des acides aminés intérieurs à la chaîne peptidique, par exemple Lys, d'autre part peuvent être modifiées, dès lors que cette modification n'entraîne pas une modification des propriétés antigéniques, le cas échéant immunogénique, de l'ensemble de la molécule. Les molécules ainsi modifiées entrent naturellement dans le cadre de la protection donnée à l'invention par les revendications. Ces fonctions carboxyles sont éventuellement acylées ou estérifiées.

D'autres modifications entrent également dans le cadre de l'invention. En particulier, les fonctions amine ou ester, ou les deux à la fois, des acides aminés terminaux peuvent être engagées elles-mêmes dans des liaisons avec d'autres acides aminés. Par exemple l'acide N-terminal peut être lié à une séquence comprenant de 1 à plusieurs acides aminés correspondant à une partie de la région C-terminale d'une autre peptide conforme à la définition qui en a été donnée plus haut, ou vice-versa.

Il va de soi également que toute séquence peptidique issue de la modification, par substitution et/ou par addition et/ou suppression d'un ou plusieurs acides aminés, d'une des séquences peptidiques décrites ci-dessus, entre dans le cadre de la protection donnée à l'invention par les revendications, dès lors que cette modification n'altère pas les propriétés antigéniques ou immunogéniques des polypeptides de l'invention, notamment lorsque ces propriétés immunogènes ont été renforcées de façon adéquate, par exemple par association de ces polypeptides avec un adjuvant immunologique approprié (par exemple un muramylpeptide) ou par couplage avec une molécule porteuse de poids moléculaire plus élevé (par exemple une sérum-albumine ou une poly-lysine) ou une toxine du type tétanique ou un autre antigène de P. falciparum.

Plus particulièrement, l'invention concerne toute séquence peptidique dérivée des séquences peptidiques sus-mentionnées, et présentant des modifications par substitution de 40 % au maximum des acides aminés tout en conservant l'activité biologique des séquences de l'invention, à savoir notamment l'induction d'une réponse des lymphocytes T, en particulier des lymphocytes T cytotoxiques.

L'invention concerne plus généralement toute molécule caractérisée par la présence dans sa structure d'une ou plusieurs séquences peptidiques présentant des réactions immunologiques croisées avec les séquences peptidiques répondant aux formules précédentes vis-à-vis des anticorps inductibles par ces dernières in vivo.

L'invention concerne également toute séquence de nucléotides codant pour un polypeptide de l'invention et plus particulièrement toute séquence de nucléotides correspondant, selon le code génétique universel, à une des séquences en acides aminés de l'invention.

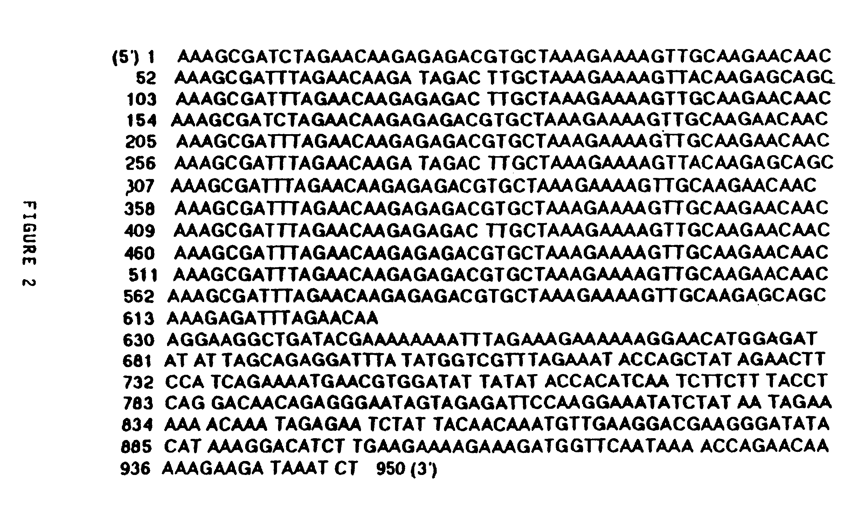

L'invention a plus particulièrement pour objet la séquence nucléotidique constituée des 951 nucléotides représentés sur la figure 2, et codant pour le polypeptide de 316 acides aminés (encore désigné ci-après par la protéine recombinante LSA-R-NR) sus-mentionné représenté sur la figure 1.

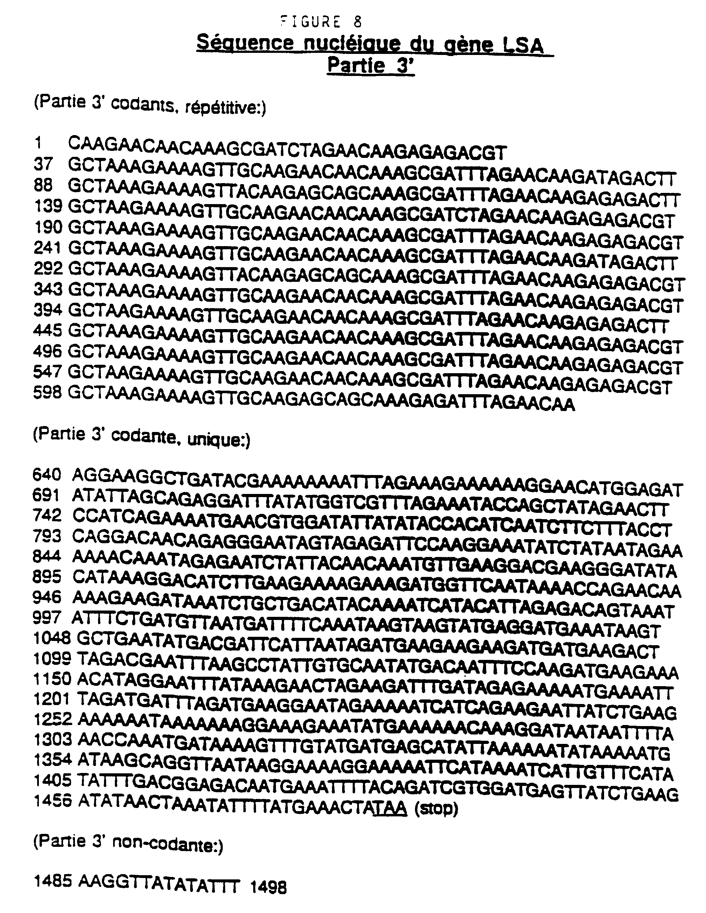

L'invention vise également la séquence nucléotidique représentée sur la figure 8, et correspondant à la partie 3' du gène LSA.

L'invention concerne aussi les séquences nucléotidiques codant pour des sous-séquences peptidiques de l'invention. on citera notamment les séquences nucléotidiques délimitées par les nucléotides situés aux positions 630 à 949, 597 à 648 (codant pour le peptide LSA-J), ou 640 à 717 (codant pour le peptide LSA-NR) de la figure 2.

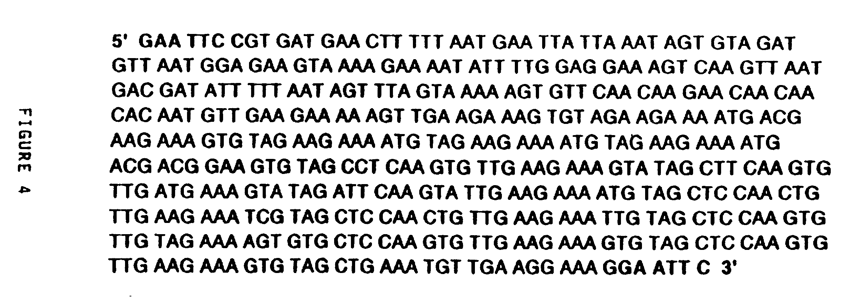

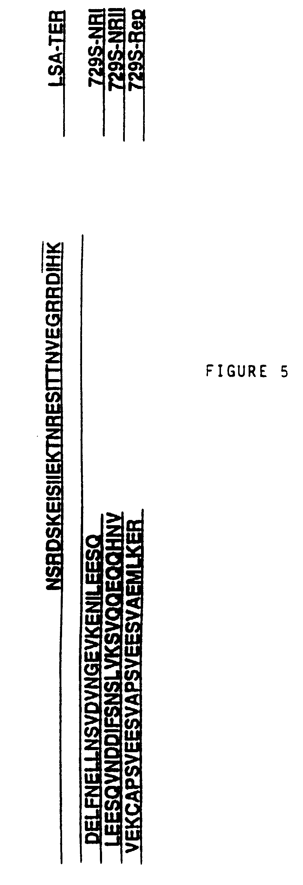

L'invention vise également toute ou partie de la séquence nucléotidique de la figure 4 codant pour toute ou partie de la séquence peptidique 729S représentée sur la figure 3.

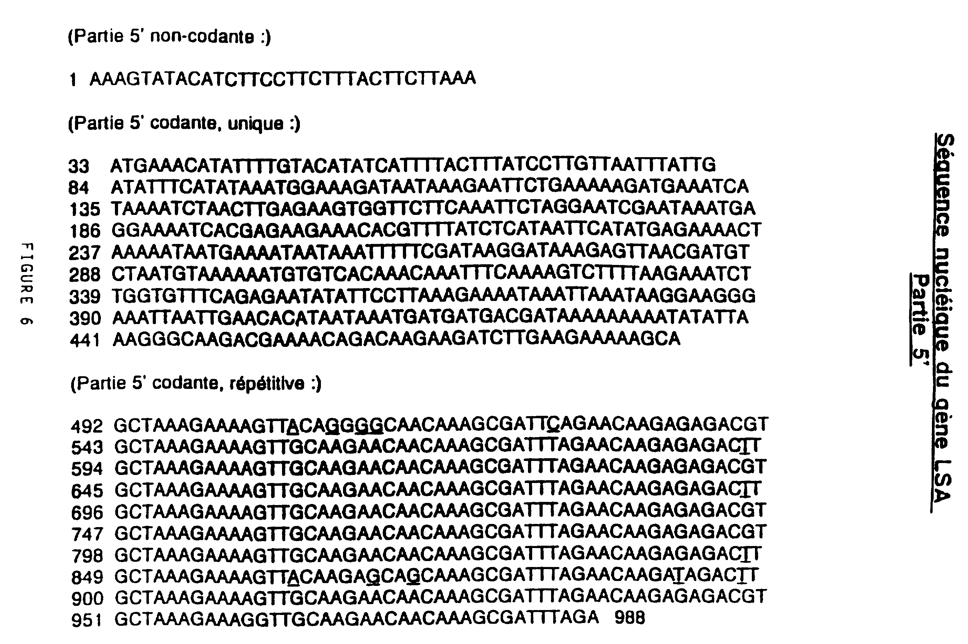

L'invention a également pour objet la séquence nucléotidique représentée sur la figure 5, et correspondant à la partie 5' du gène LSA.

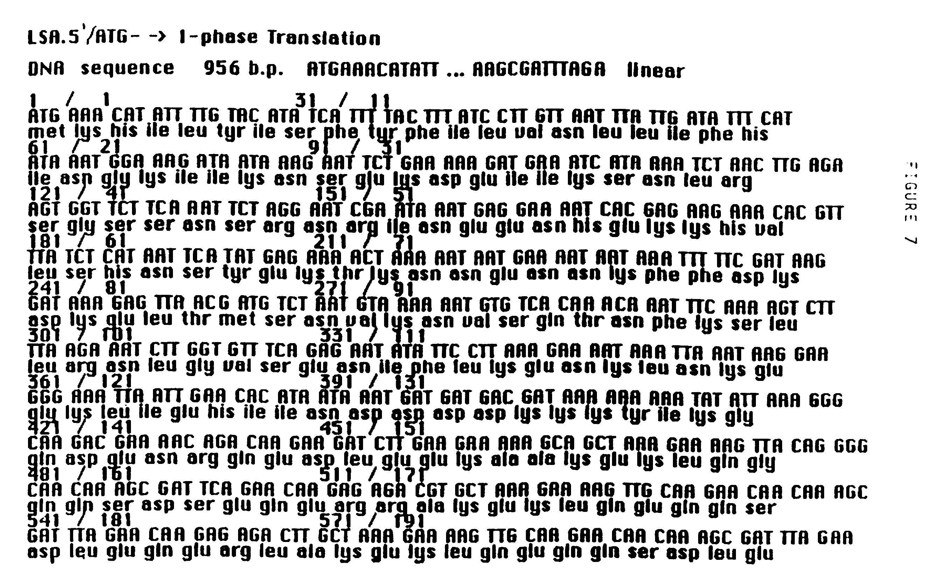

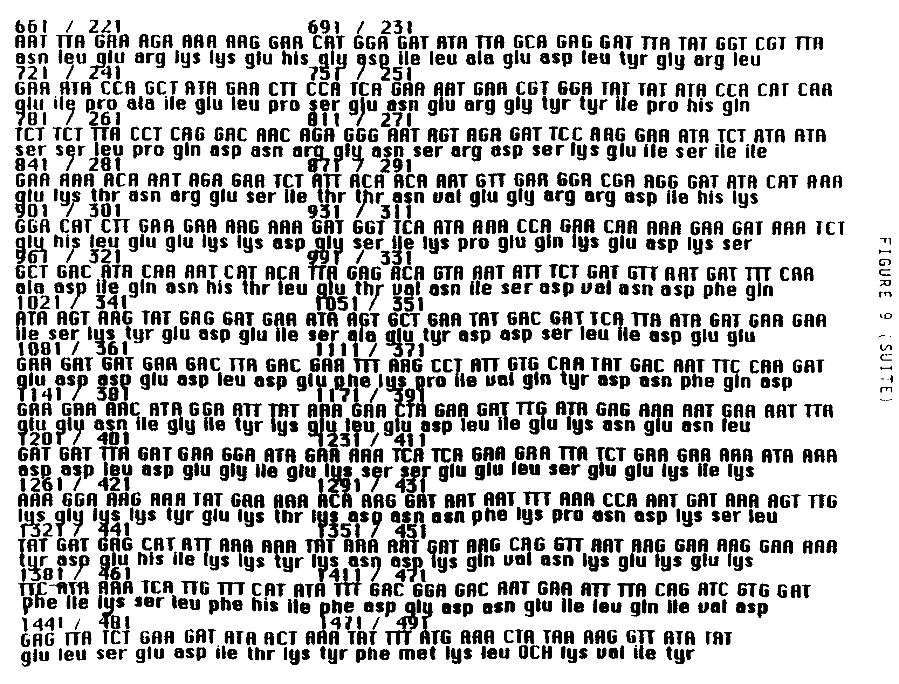

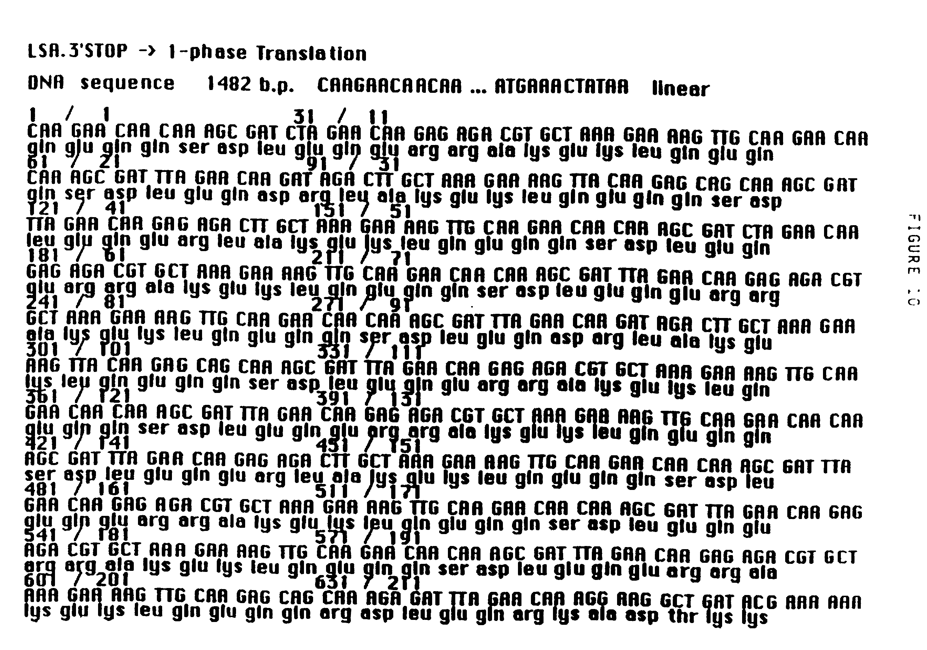

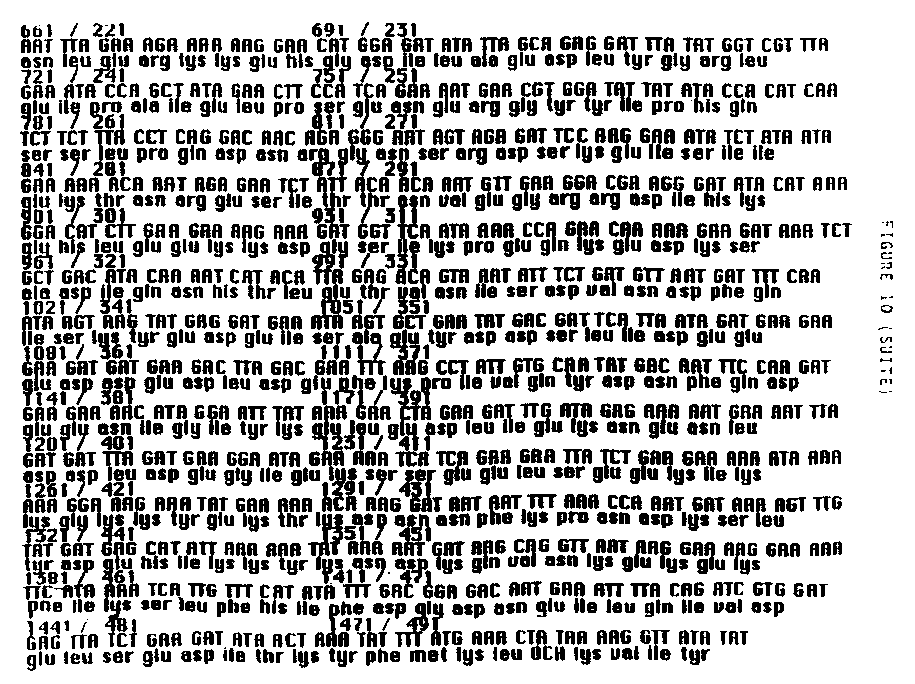

L'invention concerne également toute séquence de nucléotides codant pour un polypeptide identique, ou analogue, tant du point de vue de la structure que des caractéristiques antigéniques, à ceux de l'invention, cette séquence étant capable de s'hybrider avec tout ou partie de la séquence nucléotidique délimitée par les nucléotides situés aux positions 597 à 949 de la figure 2, ou avec tout ou partie de la séquence nucléotidique de la figure 4 ou les séquences complémentaires de ces dernières, dans les conditions suivantes :
- pré-traitement (pré-hybridation) du filtre de nitrocellulose supportant le fragment d'acide nucléique à tester avec le tampon d'hybridation, (composé de 6 SSC, 5x Deenhard's, 0,5 % SDS, 100µg/l DNA de sperme de saumon sonique dénaturé) cette opération étant effectuée à 65°C pendant 1 heure;
- remplacement du tampon d'hybridation au contact du support, sur lequel le fragment d'acide nucléique est alors fixé, par du tampon d'hybridation de même composition, et addition de la séquence sus-mentionnée de la figure 2 ou de la figure 4 en tant que sonde, notamment marquée radioactivement, et préalablement dénaturée;
- incubation dudit fragment d'acide nucléique fixé sur le support dans ce tampon d'incubation avec la séquence sus-mentionnée de la figure 2 ou de la figure 4 à 65°C pendant une durée d'environ à 1 heure;
- l'élimination du tampon contenant la sonde non fixée, par 2 lavages successifs de 30 minutes chacun avec une solution tampon composée de 2 x SSC, et 0,5 % SDS à 65°C.

Il est à rappeler que 20 x SSC = 175,3 g NaCl, 88,2 g Tricitrate de Sodium/l, PH 7; Denhard's 50 x = Ficoll 400 5 g, Polyvinil pyrrolidone 5 g, BSA (sérum albumine de boeuf) fraction V 5 g/500 ml; le SDS est le sodium dodécyl sulfate.

L'invention a également pour objet tout acide nucléique recombinant contenant au moins une séquence de nucléotides de l'invention, insérée dans un acide nucléique hétérologue vis-à-vis de ladite séquence de nucléotides.

L'invention concerne plus particulièrement un acide nucléique recombinant tel que défini ci-dessus, dans lequel la séquence de nucléotides de l'invention est précédée d'un promoteur (notamment un promoteur inductible) sous le contrôle duquel la transcription de ladite séquence est susceptible d'être effectuée et, le cas échéant, suivie d'une séquence codant pour des signaux de terminaison de la transcription.

L'invention concerne tout vecteur recombinant, utilisé en particulier pour le clonage d'une séquence nucléotidique de l'invention, et/ou l'expression du polypeptide codé par cette séquence, et caractérisé en ce qu'il contient un acide nucléique recombinant, tel que défini ci-dessus, en l'un de ses sites non essentiel pour sa réplication.

A titre d'exemple de vecteur sus-mentionné, on citera les plasmides, les cosmides, ou les phages.

A ce titre, l'invention concerne plus particulièrement le plasmide DG536 déposé à la CNCM sous le n°I-1027 le 17 janvier 1991, ainsi que le plasmide DG729S déposé à la CNCM sous le n° I-1028 le 17 janvier 1991.

L'invention a également pour objet un procédé de préparation d'un polypeptide de l'invention, par transformation d'un hôte cellulaire à l'aide d'un vecteur recombinant de type sus-indiqué, suivie de la mise en culture de l'hôte cellulaire ainsi transformé, et de la récupération du polypeptide dans le milieu de culture.

Ainsi, l'invention concerne tout hôte cellulaire transformé par un vecteur recombinant tel que défini ci-dessus, et comprenant les éléments de régulation permettant l'expression de la séquence de nucléotides codant pour un polypeptide selon l'invention.

L'invention a plus particulièrement pour objet des amorces d'ADN (ou d'ARN) utilisables dans le cadre de la synthèse de séquences nucléotidiques et/ou polypeptides selon l'invention, par la technique du PCR (Polymerase Chain Reaction) telle que décrite dans les brevets américains ne 4,683,202 et n°4,683,195 et de la demande de brevet européenne n° 200.362 (PCR = amplification en chaîne de l'ADN).

L'invention concerne toute amorce d'ADN ou d'ARN, caractérisée en ce qu'elle est constituée d'environ 10 à 25 nucléotides, identiques aux 10 à 25 premiers nucléotides de la séquence de nucléotides codant pour une séquence peptidique selon l'invention ou identiques aux 10 à 25 derniers nucléotides de ladite séquence.

L'invention concerne également toute amorce d'ADN ou d'ARN, caractérisée en ce qu'elle est constituée d'environ 10 à 25 nucléotides complémentaires des 10 à 25 premiers nucléotides de la séquence nucléotidique codant pour une séquence peptidique selon l'invention ou complémentaire des 10 à 25 derniers nucléotides de ladite séquence de nucléotides.

L'invention a également pour objet toute amorce d'ADN ou d'ARN, caractérisée en ce qu'elle est constituée d'environ 10 à 25 nucléotides capables de s'hybrider avec les 10 à 25 premiers nucléotides ou avec les 10 à 25 derniers nucléotides de ladite séquence de nucléotides codant pour un polypeptide de l'invention, dans les conditions d'hybridation définies ci-dessus.

Ainsi la présente invention concerne plus particulièrement un procédé de préparation d'un polypeptide de l'invention comprenant les étapes suivantes;
- le cas échéant, l'amplification préalable suivant la technique PCR de la quantité de séquences de nucléotides codant pour ledit polypeptide à l'aide de deux amorces d'ADN choisies de manière à ce que l'une de ces amorces soit identique aux 10 à 25 premiers nucléotides de la séquence nucléotidique codant pour ledit polypeptide, tandis que l'autre amorce est complémentaire des 10 à 25 derniers nucléotides (ou s'hybride avec ces 10 à 25 derniers nucléotides) de ladite séquence nucléotidique, ou inversement de manière à ce que l'une de ces amorces soit identique aux 10 à 25 derniers nucléotides de ladite séquence, tandis que l'autre amorce est complémentaire des 10 à 25 premiers nucléotides (ou s'hybride avec les 10 à 25 premiers nucléotides) de ladite séquence nucléotidique, suivie de l'introduction desdites séquences de nucléotides ainsi amplifiées dans un vecteur approprié,
- la mise en culture, dans un milieu de culture approprié, d'un hôte cellulaire préalablement transformé par un vecteur approprié contenant un acide nucléique selon l'invention comprenant la séquence nucléotidique codant pour ledit polypeptide, et
- la récupération, à partir du susdit milieu de culture du polypeptide produit par ledit hôte cellulaire transformé.

A titre d'exemple d'amorces d'ADN ou d'ARN selon l'invention, on citera les séquences suivantes:

3'->5 : TTTCGCTAGATCTTGTT et TCTAAATAGAAGAAA

Les peptides selon l'invention peuvent également être préparés par les techniques classiques, dans le domaine de la synthèse des peptides. Cette synthèse peut être réalisée en solution homogène ou en phase solide.

Par exemple, on aura recours à la technique de synthèse en solution homogène décrit par HOUBENWEYL dans l'ouvrage intitulé "Meuthode der Organischen Chemie" (Méthode de la Chimie Organique) édité par E. Wunsch, vol. 15-I et II., THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment aprés activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide.

Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'& l'acide aminé N-terminal.

Selon une autre technique préférée de l'invention, on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherchée, à partir du résidu amino-acyle C-terminal sur la fonction amine déprotégée du premier acide aminé C-terminal fixé sur la chaîne. De péférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acide aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acide aminés constituant la chaîne peptidique et on détache le peptide de la résine par exemple à l'aide d'acide fluorhydrique.

L'invention concerne également les oligoméres hydrosolubles des peptides monomères sus-indiqués.

L'oligomérisation peut provoquer un accroissement de l'immunogénicité des peptides monomères selon l'invention. Sans qu'une telle indication chiffrée puisse être considérée comme limitative, on mentionnera néanmoins que ces oligomères peuvent, par exemple, contenir de 2 à 10 unités monomères.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère contenant le nombre de motifs monomères requis pour l'acquisition de l'immunogénicité désirée.

Une méthode d'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétérobifonctionnels.

L'invention concerne encore les conjugués obtenus par couplage covalent des peptides selon l'invention (ou des susdits oligomères) à des molécules porteuses (naturelles ou synthétiques), physiologiquement acceptables et non toxiques, par l'intermédiaire de groupements réactifs complémentaires respectivement portés par la molécule porteuse et le peptide. Des exemples de groupements appropriés sont illustrés dans ce qui suit :
A titre d'exemple de molécules porteuses ou supports macromoléculaires entrant dans la constitution des conjugués selon l'invention, on mentionnera des protéines naturelles, telles que l'anatoxine tétanique, l'ovalbulmine, des sérums albumines, des hémocyamines, le PPD de la tuberculine (PPD : "Purified Protein Derivative"), etc...
A titre de supports macromoléculaires synthétiques, on mentionnera par exemple des polylysines ou des poly(D-L-alanine)-poly(L-lysine).

La littérature mentionne d'autres types de supports macromoléculaires susceptibles d'être utilisés, lesquels présentent en général un poids moléculaire supérieur à 20 000.

Pour synthétiser les conjugués selon l'invention, on peut avoir recours à des procédés connus en soi, tels que celui décrit par FRANTZ et ROBERTSON dans Infect. and Immunity, 33, 193-198 (1981), ou celui décrit dans Applied and Environmental Microbiology, (octobre 1981), vol. 42, n° 4, 611-614 par P.E. KAUFFMAN en utilisant le peptide et la molécule porteuse appropriée.

Dans la pratique, on utilisera avantageusement comme agent de couplage les composés suivants, cités à titre non limitatif : aldéhyde glutarique, chloroformiate d'éthyle, carbodiimides hydrosolubles [N'(3-diméthylamino-propyl) carbodiimide, HCl], diisocyanates, bis-diazobenzidine, di- et trichloro-s-triazines, bromures de cyanogène, ainsi que les agents de couplage mentionnés dans Scand. J. Immunol., (1978), vol. 8, p. 7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives de molécules supports. Avantageusement, il s'agit des fonctions carboxyle et amine, lesquelles peuvent donner lieu à une réaction de couplage en présence d'un agent de couplage du genre de ceux utilisés dans la synthése des protéines, par exemple,le 1-éthyl-3-(3-diméthylaminopropyl)-carbodiximide, le N-hydroxybenzotriazole, etc... On peut encore avoir recours à la glutaraldéhyde, notamment lorsqu'il s'agit de relier entre eux des groupes aminés respectivement portés par le peptide et la molécule support.

Les acides nucléiques de l'invention peuvent être préparés soit par un procédé chimique, soit par d'autres procédés.

Un mode de préparation approprié des acides nucléiques comportant au maximum 200 nucléotides (ou 200 pb, lorsqu'il s'agit d'acides nucléiques bicaténaires) de l'invention comprend les étapes suivantes :
- la synthèse d'ADN en utilisant la méthode automatisée des β-cyanethylphosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée. Un mode de préparation, par voie chimique, d'acides nucléiques de longueur supérieure à 200 nucléotides (ou 200pb, lorsqu'il s'agit d'acides nucléiques bicaténaires) de l'invention comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

Les acides nucléiques de l'invention peuvent également être préparés de la manière suivante :
- incubation de l'ADN génomique, isolé à partir d'une souche de P. falciparum, avec de l'ADNase I, puis addition d'EDTA et purification par extraction au mélange phenol/chloroforme/alcool isoamylique (25/24/1) puis par l'éther,
- traitement de l'ADN ainsi extrait par de l'Eco R1 méthylase en présence de DTT, et purification par extraction telle que décrite ci-dessus,
- incubation de l'ADN ainsi purifié avec les 4 désoxynucléotides triphosphates dATP, dCTP, dGTP, et dTTP en présence de T4 ADN polymérase et d'ADN ligase de E. coli, puis purification selon la méthode décrite ci-dessus,
- le clonage des acides nucléiques ainsi obtenus dans un vecteur approprié et la récupération de l'acide nucléique recherché à l'aide d'une sonde appropriée.

Les sondes nucléotidiques utilisées pour la récupération de l'acide nucléique recherché dans les procédés sus-mentionnés, sont constituées généralement de 40 à 200 nucléotides de la séquence nucléotidique représentée sur la figure 2 (plus particulièrement choisis parmi ceux situés entre les positions 597 à 949 de la figure 2) ou sur la figure 4, ou sa séquence complémentaire, et sont susceptibles de s'hybrider avec l'acide nucléique recherché dans les conditions d'hybridation définies ci-dessus. La synthèse de ces sondes est effectuée selon la méthode automatisée des β-cyanethylphosphoramidite décrite dans Bioorganic Chemistry 4, 274-325 (1986).

Les molécules selon l'invention possèdent des propriétés antigéniques caractéristiques des antigènes porteurs d'épitopes T, et le cas échéant B, et qui sont soit spécifiques du stade hépatique du développement de P. falciparum, soit spécifiques des stades sporozoïte, hépatique et sanguin à la fois.

En effet, comme il le sera plus particulièrement dècrit à l'aide d'exemples de molécules selon l'invention dans la description détaillée qui suit, les molécules selon l'invention comportant tout ou partie de l'enchaînement des acides aminés compris entre les positions 200 et 316 de la figure 1, réagissent spécifiquement avec les anticorps ou les lymphocytes dirigés contre les épitopes B et/ou T des antigènes produits au stade hépatique de P. falciparum, mais pas avec les anticorps dirigés contre d'autres antigènes produits par P. falciparum ou contre des antigènes produits par d'autres espèces de Plasmodium.

Ces molécules selon l'invention reconnaissent donc spécifiquement les anticorps produits par le système immunitaire d'un individu infecté par P. falciparum sous l'effet de l'antigène LSA dont le caractère fortement immunogène a été précédemment mentionné.

Les molécules selon l'ivention comprenant tout ou partie de l'enchaînement peptidique de la figure 3 ne sont pas reconnus par les anticorps précédents réagissant spécifiquement avec tout ou partie du polypeptide délimité par les acides aminés situés aux positions 200 à 316 de la figure 1.

Par contre les polypeptides correspondant à tout ou partie de l'enchaînement peptidique de la figure 3 sont reconnus par des anticorps réagissant spécifiquement avec des antigènes localisés à la surface de sporozoïtes (provenant de souches différentes de P. falciparum), ainsi qu'avec des antigènes des schizontes hépatiques et des schizontes sanguins, et enfin avec la surface des sporozoïtes de P. yoelii mais pas de P. Berghei.

Il faut souligner également que les anticorps reconnaissant spécifiquement les polypeptides correspondant à tout ou partie de l'enchaînement peptidique de la figure 3 sont capables de bloquer à 100 % l'entrée des sporozoïtes de P. yoelii dans des cellules hépatiques de rongeurs in vitro, à la différence des anticorps dirigés contre la protéine circumsprozoïtaire de P. yoelii, comme de P. falciparum.

La possibilité de production en grande quantité des molécules selon l'invention ainsi que leurs propriétés de reconnaissance spécifique d'anticorps parmi les plus activement produits lors de l'infection d'un individu par P. falciparum, font desdites molécules des réactifs de choix pour le diagnostic in vitro du paludisme chez un individu infecté par P. falciparum.

L'invention concerne donc un procédé de détection in vitro d'anticorps corrélables au paludisme issu de l'infection d'un individu par P. falciparum dans un tissu ou fluide biologique susceptible de les contenir, ce procédé comprenant la mise en contact de ce tissu ou fluide biologique avec une molécule selon l'invention dans des conditions permettant une réaction immunologique in vitro entre lesdites molécules et les anticorps éventuellement présents dans le tissu ou fluide biologique, et la détection in vitro des complexes antigènes-anticorps éventuellement formés.

De préférence, le milieu biologique est constitué par un sérum humain.

Toute procédure classique peut être mise en oeuvre pour réaliser une telle détection.

A titre d'exemple une méthode préférée met en jeu des processus immunoenzymatiques selon la technique ELISA, ou immunofluorescents, ou radioimmunologiques (RIA) ou équivalent.

Ainsi l'invention concerne également toute molécule décrite ci-dessus marquée à l'aide d'un marqueur adéquat du type enzymatique, fluorescent, radioactif, etc...

De telles méthodes comprennent par exemple les étapes suivantes :
- dépôt de quantités déterminées d'une composition polypeptidique selon l'invention dans les puits d'une microplaque de titration,
- introduction dans lesdits puits de dilutions croissantes du sérum devant être diagnostiqué,
- incubation de la microplaque,
- rinçages répétés de la microplaque,
- introduction dans les puits de la microplaque d'anticorps marqués contre des immunoglobulines du sang, le marquage de ces anticorps ayant été réalisé à l'aide d'une enzyme sélectionnée parmi celles qui sont capables d'hydrolyser un substrat en modifiant l'absorption des radiations de ce dernier, au moins à une longueur d'onde déterminée,
- détection, en comparaison avec un témoin de contrôle, de la quantité de substrat hydrolysé.

L'invention concerne également des coffrets ou kits pour le diagnostic in vitro du paludisme provoqué par P. falciparum qui comprennent :
- une composition polypeptidique selon l'invention,
- les réactifs pour la constitution du milieu propice à la réalisation de la réaction immunologique,
- les réactifs permettant la détection des complexes antigènes-anticorps produits par la réaction immunologique, ces réactifs pouvant également porter un marqueur, ou être susceptibles d'être reconnus à leur tour par un réactif marqué, plus particulièrement dans le cas où la composition polypeptidique sus-mentionnée n'est pas marquée,
- un tissu ou fluide biologique de référence dépourvu d'anticorps reconnus par la composition polypeptidique sus-mentionnée.

L'invention concerne les anticorps eux-mêmes formés contre les polypeptides de l'invention.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention, d'une part et des cellules d'une lignée de cellules d'un myélome approprié d'autre part, et d'être sélectionné, par sa capacité à produire des anticorps monoclonaux reconnaissant le polypeptide initialement mis en oeuvre pour l'immunisation des animaux.

L'invention vise également une méthode de diagnostic in vitro du paludisme chez un individu susceptible d'être infecté par P. falciparum qui comprend la mise en contact d'un tissu ou d'un fluide biologique prélevé chez un individu avec des anticorps tels que décrits ci-dessus, dans des conditions permettant une réaction immunologique in vitro entre lesdits anticorps et les protéines spécifiques de P. falciparum éventuellement présentes dans le tissu biologique, et la détection in vitro des complexes antigènes-anticorps éventuellement formés.

A ce titre l'invention a pour objet un coffret ou kit pour le diagnostic in vitro du paludisme comprenant :
- des anticorps tels que décrits ci-dessus,
- les réactifs pour la constitution du milieu propice à la réalisation de la réaction immunologique,
- les réactifs permettant la détection des complexes antigènes-anticorps produits par la réaction immunologique, ces réactifs pouvant également porter un marqueur, ou être susceptibles d'être reconnus à leur tour par un réactif marqué, plus particulièrement dans le cas où les anticorps sus-mentionnés ne sont pas marqués.

L'invention concerne également une sonde nucléotidique de détection caractérisée en ce qu'elle est constituée par tout ou partie d'une des séquences de nucléotides telles que définies ci-dessus de l'invention.

L'invention a plus particulièrement pour objet une méthode de diagnostic in vitro du paludisme chez un individu susceptible d'être infecté par P. falciparum qui comprend les étapes suivantes :
- éventuellement l'amplification préalable de la quantité de séquences de nucléotides selon l'invention, susceptibles d'être contenues dans l'échantillon biologique prélevé chez ledit individu, à l'aide de deux amorces d'ADN choisies de la manière indiquée ci-dessus,
- la mise en contact de l'échantillon biologique sus-mentionné avec une sonde nucléotidique telle que définie ci-dessus, dans des conditions permettant la production d'un complexe d'hybridation formé entre ladite sonde et ladite séquence de nucléotides,
- la détection du complexe d'hybridation sus-mentionné éventuellement formé.

A tire d'exemple de sondes nucléotidiques de l'invention, on citera les séquences suivantes :

3'->5' : TTTCGCTAGATCTTGTT et TCTAAATAGAAGAAA

L'invention ouvre surtout la voie à la mise au point de nouveaux principes vaccinants contre le paludisme issu de l'infection d'un individu par P. falciparum.

L'invention concerne également les compositions préparées sous forme de vaccins contenant soit un ou plusieurs peptides selon l'invention, soit un oligomère de ce ou ces peptides, soit encore un conjugué de ce ou ces peptides ou oligomère avec une molécule porteuse, en association avec un véhicule pharmaceutiquement acceptable approprié et, le cas échéant, avec d'autres principes actifs vaccinants contre le paludisme.

Une composition pharmaceutique particulièrement avantageuse, de l'invention est caractérisée en ce qu'elle comprend tout ou partie de l'enchaînement peptidique délimité par les acides aminés situés aux positions 200 à 316 dé la figure 1, en association avec tout ou partie de la séquence peptidique représentée sur la figure 3.

Des compositions pharmaceutiques avantageuses sont constituées par des solutions, suspensions ou liposomes injectables contenant une dose efficace d'au moins un produit selon l'invention. De préférence, ces solutions, suspensions ou liposomes sont réalisés dans une phase aqueuse stérilisée isotonique, de préférence saline ou glucosée.

L'invention concerne plus particulièrement de telles suspensions, solutions ou forme liposome qui sont aptes à être administrées par injections intradermiques, intramusculaires ou sous-cutanées, ou encore par scarifications.

Elle concerne également des compositions pharmaceutiques administrables par d'autres voies, notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des compositions pharmaceutiques dans lesquelles l'un au moins des produits selon l'invention se trouve associé à des excipients pharmaceutiquement acceptables, solides ou liquides, adaptés à la constitution de formes orales, oculaires ou nasales, ou avec des excipients adaptés à la constitution des formes d'administration rectale, ou encore avec des excipients gélatineux pour l'administration vaginale. Elle concerne aussi des compositions liquides isotoniques contenant l'un au moins des conjugués selon l'invention, adaptées à l'administration sur les muqueuses, notamment oculaires ou nasales.

Avantageusement, les compositions vaccinales selon l'invention contiennent en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthylcellulose, les hydroxydes et phosphates d'aluminium, la saponine ou tous autres adjuvants de ce type, bien connus de l'homme de l'art. Enfin elles contiennent si besoin un adjuvant immunologique, notamment du type muramylpeptide.

L'invention concerne aussi des compositions pharmaceutiques contenant à titre de substance active l'un au moins des anticorps polyclonaux ou monoclonaux définis précédemment en association avec un véhicule pharmaceutiquement acceptable.

L'invention ne se limite évidemment pas aux modes de réalisation décrits ci-dessus à titre d'exemples et l'homme de l'art peut y apporter des modifications sans pour autant sortir du cadre des revendications ci-après ; notamment certains des acides aminés intervenant dans la séquence des peptides selon l'invention peuvent être remplacés par des acides aminés isofonctionnels ou isostériques ; par exemple, une ou plusieurs des substitutions suivantes peuvent être envisagées :
- Glu est substitué par Asp ou Gln,
- Leu est remplacé par Ala, etc...

Il est naturellement bien entendu que les peptides qui résultent de telles substitutions consistent en des équivalents des peptides plus particulièrement revendiqués, dès lors qu'eux-mêmes ou des oligomères ou conjugués formés à partir de ces peptides présentent des propriétés immunogènes semblables.

L'invention concerne également encore plus particulièrement les "protéines chimères" qui peuvent être obtenues par les techniques du génie génétique, ces protéines chimères pouvant contenir une ou plusieurs des séquences peptidiques de l'invention, et incorporées ou rattachées à un fragment peptidique autre que la β-galactosidase. Ce dernier fragment peptidique a de préférence un poids moléculaire suffisant pour renforcer l'immunagénicité des séquences peptidiques selon l'invention et n'interfère pas du point de vue immunologique avec la manifestation de l'immunogénicité recherchée.

Des caractéristiques supplémentaires de l'invention apparaîtront encore au cours de la description qui suit des conditions dans lesquelles les polypeptides de l'invention ont été obtenus.

Des sérums provenant d'individus européens vivant dans des zones endémiques et suivant une prophylaxie continue avec des médicaments dirigés contre les schizontes des stades sanguins (chloroquine) ont été sélectionnés et testés en utilisant des antigènes du stade sporozoïte, du stade hépatique (antigène LS), et des stades sanguins. La plupart de ces sérums réagissent avec les antigènes de tous les stades probablement car la prophylaxie a été interrompue. Trois sérums prélevés à partir d'individus ayant résidé en Afrique tropicale rurale et ayant ingéré 100 µg de chloroquine par jour sans interruption pendant 23 à 26 ans, ne réagissent pas avec les antigènes des stades sanguins suivant le test d'immunofluorescence (IFA). Ces trois sérums possédant toutefois des titres élevés en anticorps dirigés contre les sporozoïtes et les protéines LS (dilution IFA 1/3200 et 1/6400 respectivement).

Un des trois sérums précédents, de spécificité réduite, a été utilisé pour cribler une banque d'ADN génomique construite dans le bactériophage λgt 11 de la manière suivante :

### 1) CONSTRUCTION DE LA BANQUE D'ADN GENOMIQUE DE Plasmodium falciparum.

L'ADN génomique du clone 96 de la souche thailandaise Tak9 de P. falciparum (Science, 212, 1.37-1.38 (1981) a été isolé par les techniques classiques.

Des échantillons de 18 µg d'ADN de P. falciparum ont été incubés à 15°C dans un tampon 50 mM Tris HCl pH 7.5, 1 mM MnCl₂, 20 µg/ml de sérum albumine bovine, avec des quantités respectives d'ADNase I (Boehringer Mannheim) de 5 pg pendant 5 minutes ou bien de 3.5 pg pendant 5 ou 10 minutes. Après addition de 5 mM EDTA (Ethylènediamine tetracétique acide), les échantillons d'ADN sont réunis et purifiés par extraction au mélange phénol/chloroforme/alcool isoamylique (25 V/24 V/1 V) puis par l'éther. L'ADN est concentré par précipitation à l'éthanol à -20.C en présence de 2.5 M d'acétate d'ammonium.

45 µg d'ADN ainsi traité ont été méthyles par 180 U d'Eco R1 méthylase (Biolabs) dans les conditions recommandées par le fournisseur, avec l'addition supplémentaire de 5 mM DTT (dithiothreitol), pendant 13 minutes à 37°C. Après purification de l'ADN comme ci-dessus, 10 µg d'ADN ont été incubés avec 40 mM Tris HCl pH 8.0, 10 mM sulfate d'ammonium, 10 mM 2-mercaptoethanol, 0.5 m M EDTA, 0.05 mM NAD (nicotinamide adénine dinucléotide) 0.1 mM dXTP (comprenant les 4 desoxynucleotides triphosphates dATP, dCTP, dGTP et dTTP), en présence de 10 U T4 ADN polymérase (PL Biochemicals) et de 10 U de E. coli ADN ligase (Biolabs). L'ADN a été purifié et concentré comme ci-dessus.

8 µg d'ADN ont alors été ligaturés avec 0.4 µg d'un adaptateur ou "linker" Eco R1 (adaptateurs phosphorylés Eco R1 commercialisés par Biolabs) par 4 U T4 ADN ligase (Biotec) dans le tampon 50 mM Tris HCl pH 8.0, 10 mM MgCl₂, 20 mM DTT, 1 mM ATP, 50 µg/ml sérum albumine bovine.

Aprés incubation à 4°C pendant 5 heures, on ajoute 2 U T4 ADN ligase et la réaction est poursuivie à 4°C pendant 16 heures. Le tube est soumis à plusieurs cycles de congélation à -80°C/décongélation pour arrêter la réaction. L'ADN est ensuite dilué et le tampon d'incubation ajusté de façon à obtenir les conditions recommandées par le fournisseur pour l'utilisation de l'enzyme Eco R1. 100 U d'enzyme Eco R1 (Promega Biotec) sont ajoutées et incubées pendant 3 heures à 37°C. La réaction est arrêtée par un chauffage de 10 minutes à 60°C, et l'ADN est purifié et concentré comme ci-dessus.

L'ADN est resuspendu dans 100 µl de tampon 50 mM Tris HCl pH 8.0, 1 mM EDTA, et déposé sur un gradient 5-20 % de saccharose préparé en 25 mM acétate de sodium, 10 mM EDTA et centrifugé dans le rotor Beckman SW 50.1 à 45,000 tours par minute pendant 150 minutes.

Les fractions sont analysées sur gel d'agarose et celles qui contiennent les fragments d'ADN de tailles comprises entre environ 300 pb. et 2 500 pb. sont rassemblées, dialysées contre le tampon 50 mM Tris HCl pH 8.0, 1 mM EDTA à 4°C. L'ADN est concentré par précipitation à l'éthanol. Environ 400 ng de cet ADN ont été ligaturés à 1 µg d'ADN du vecteur λgt11 (Proc. Natl. Acad. Sci., USA, 80, 1194-1198 (1983)) coupé par Eco R1 et déphosphorylé (Protoclone de Promega Biotec), dans un volume de 10 µl, (en tampon 50 mM Tris HCl pH 8.0, 10 mM MgCl₂, 20 mM DTT, 1 mM ATP, 50 µg/ml sérum albumine bovine) par 1 U T4 ADN ligase (Biotec).

Les produits de ligature ont été encapsidés in vitro dans les extraits d'E. coli préparés à partir des souches bactériennes construites par B. Hohn (Methods Enzymol. 68, 299), selon la technique décrite par Maniatis et coll. (Molecular cloning, a laboratory manual, p. 264, Cold Spring Harbor Laboratory (1982)).

Environ 7 millions de bactériophages recombinants ont été obtenus.

### 2) CRIBLAGE IMMUNOLOGIQUE DE LA BANQUE

Les bactériophages recombinants ont été étalés sur un milieu de culture contenant la bactérie indicatrice Y 1090, à une densité de 50,000 plages par boite de Pétri de 90 mm, et incubés à 42°C pendant 3 heures.

Un filtre de nitrocellulose (Schleicher & Schuell, BA 85) saturé par 0,01 M IPTG isopropyl-b-thiogalactopyranoside (Sigma) est déposé sur les boites, qui sont incubées à 37°C pendant 3 heures. Au terme de ces incubations, les filtres de nitrocellulose sont prélevés, et les boites de Pétri conservées à 4°C.

Les filtres de nitrocellulose sont placés dans un bain de tampon TL : 50 mM tris HCl pH 8.0, 150 mM NaCl, 5 % lait écrémé, 0.05 % Tween 20 (Sigma). Les filtres sont incubés 15 heures à 4°C en tampon TL, puis 2 fois 15 minutes à 20°C. Ils sont alors incubés pendant une heure avec un pool d'antisérums humains immuns dirigés contre les antigènes de tous les stades de développement de P. falciParum, traité au préalable pour le dépléter en anticorps anti-E.coli selon la technique décrite par Ozaki et coll. (J. Immunol. Methods, 89, 213-219, 1986). Le pool d'antisérums humains a été utilisé à la dilution 1/200, en tampon TL. L'incubation a été faite à 20°C pendant 1 heure. Les filtres ont été lavés 4 fois avec le tampon TL, puis incubés avec des anticorps anti-immunoglobulines humaines conjugués à la peroxydase de raifort (Biosys) et iodinés à l'iode 125I, pendant 1 heure à 20°C. Aprés plusieurs lavages en tampon TL, puis en tampon 50 mM Tris HCl pH 8.0, 150 mM NaCl, l'activité enzymatique de la peroxydase est révélée (Ozaki et coll, précédemment cité), les filtres sont séchés à l'air libre et autoradiographies sur film Kodak Royal X-OMat AR, avec un écran amplificateur.

Une collection d'environ 1 200 clones de bactériophages recombinants a été constituée en prélevant les plages de lyse correspondant aux signaux positifs. Ces clones ont été ensuite soumis à un second cycle de criblage immunologique, en employant cette fois un des trois sérums humains précédemment décrits et présentant pas ou peu d'anticorps dirigés contre les formes érythrocytaires de P. falciparum et un titre élevé contre les formes sporozoïte et hépatique du parasite (formes pré-érythrocytaires). Ce criblage immunologique a été effectué selon le protocole décrit ci-dessus. Ce sérum a conduit à identifier environ 120 clones recombinants parmi 1200 testés.

Les anticorps humains qui réagissent avec les déterminants antigéniques exprimés par les clones recombinants ont été purifiés par affinité sur les protéines recombinantes, selon la technique décrite par Ozaki et coll. (précédemment cité). Ces anticorps spécifiques ont été incubés avec des préparations de parasites à différents stades de développement (sporozoite, stade hépatique ou stades érythrocytaires), et la réaction a été étudiée par immunofluorescence indirecte.

Les clones recombinants sur lesquels sont retenus par affinité des anticorps spécifiques du stade hépatique, et donc qui expriment des déterminants propres à ce stade ont été étudiés : il s'agit des clones DG 307, DG 199 et DG 145. Ces anticorps spécifiques de ces trois clones réagissent spécifiquement avec les schizontes hépatiques, tels que l'on peut les obtenir après infection d'hépatocytes humains ou de singes par des sporozoïtes de P. falciparum; la localisation de la fluorescence a été déterminée comme étant identique à celle considérée caractéristique de LSA.

La spécificité d'espèce et de stade des 3 clones DG 145, DG 199, et DG 307 a été testée de la manière suivante. Premièrement, il a été déterminé que les mêmes anticorps purifiés par affinité et qui réagissent par IFA (ou encore qui sont IFA positifs) avec LSA, ne réagissent pas avec des préparations de sporozoïtes sèches ou humides, ni avec les antigènes des stades sanguins, qu'ils soient testés par IFA avec des parasites fixés à l'acétone, ou par immunotransfert (immunoblotting) en utilisant des protéines de tous les stades extraites au SDS. Les anticorps purifiés par affinité ne réagissent pas avec les antigénes de stade hépatique de P. yoelii, ni avec les schizontes hépatiques de P. vivax préparés à partir de singes Saimiri sciureus.

Deuxièmement, les protéines recombinantes de DG 145, DG 199 et DG 307 ne réagissent pas avec les sérums provenant de deux patients atteints de malaria (paludisme causé par P. falciparum) par transfusion accidentelle et qui, par définition n'ont donc pas d'anticorps contre les antigènes spécifiques des stades précédents (sporozoïtes et antigènes du stade hépatique). Ces protéines ne réagissent pas avec deux anticorps monoclonaux reconnaissant le tétrapeptide CS, avec les sérums de souris immunisées avec les antigènes CS recombinants R32t et 32 (Science, 228, 958 (1985)). De plus, les protéines recombinantes n'ont pas réagi avec des antisérums humains dirigés contre P. vivax (bien que les sérums furent positifs avec les schizontes hépatiques de P. vivax), P. ovale et P. cynomolgi (Ann. Soc. Belg. Med. Trop., 60, 348 (1980)) quand elles sont testées par la technique de taches d'immunotransfert (immunodot blots), alors qu'elles sont positives avec tous les sérums humains anti-P. falciparum testés.

Au sein de cette sous-population de 120 clones sus-mentionnée, une technique de criblage immunologique par des anticorps purifiés par affinité sur le clone DG307 (ou 145, ou 199) conduit à la détection de 40 clones environ sur 120 présentant apparemment l'épitope caractéristique du LSA et défini par la structure de base de 17 acides aminés citée plus haut.

De même des techniques d'identification par hybridation à l'aide de fragments d'ADN des mêmes clones (DG307) permettent d'identifier ces structures répétées dans les mêmes clones que ceux identifiés par les tests immunologiques.

Un criblage ccmplémentaire du sous-ensemble de 40 clones appartenant à cette famille de l'antigène LSA a été utilisé pour identifier d'autres parties du gène comportant des séquences distinctes de celles définies par les répétitions de 17 acides aminés. Pour ce criblage complémentaire, des sérums identifiés comme ne réagissant pas avec les répétitions des 17 acides aminés, mais positifs en immunofluorescence indirecte avec la structure périphérique du schizonte hépatique où est situé l'antigène LSA, ont été employés. Au sein de la famille des 40 clones du LSA, ces sérums ont été trouvés positifs pour plusieurs d'entre eux, et l'un des clones comportant le plus grand insert, dénommé DG536, a été sélectionné et étudié en détail.

D'autres clônes, DG538, DG750 et DG443 ont été étudiés. Les clônes DG750 et DG443 contiennent la majeure partie de la séquence 5' non répétitive du gène LSA.

L'insert de 951 paires bases a été purifié et recloné dans le bactériophage M13 mp19. La séquence d'ADN et l'organisation génomique du gène LSA ont alors été déterminées. La figure 1 montre que le clone comporte à l'extrémité 5' une séquence de 209 acides aminés correspondant à une série des 12 répétitions de 17 acides aminés, similaire à celle décrite dans l'article de Guérin-Marchand et coll. (Nature, sus-mentionné) et comporte ensuite une série de 106 acides aminés dont la structure est non répétitive.

Ainsi qu'on le voit dans la figure 1, le motif de 17 acides aminés est dans deux répétitions (cf. motif correspondant aux positions 35 à 51, et celui correspondant aux positions 137 à 153 de la figure 1) identique à celui décrit dans l'article de Guérin-Marchand et coll. et les autres répétitions présentent une substitution d'une leucine par une arginine (cf. positions 8, 59, 76, 110, 127, 161, 178 et 195 de la figure 1), une substitution d'un acide glutamique par un acide aspartique (cf. positions 23 et 91 de la figure 1), ainsi qu'une substitution d'une sérine par une arginine (cf. position 205 de la figure 1).

La taille de la protéine native du LSA dans le parasite a pu être mesurée au prix de grandes difficultés. Des schizontes hépatiques de P. falciparum ont été produits in vitro par infection d'hépatocytes humains de primo culture avec des glandes salivaires de moustiques contenant des sporozoites selon la technique décrite dans Mazier et coll. (Science n°227, p440, 1985). Après 7 jours de culture, les cellules infectées sont recueillies et utilisées pour préparer un extrait analysé en gel de polyacrylamide contenant du SDS et transféré sur nitrocellulose. Les antigènes hépatiques sont ensuite révélés par un anticorps purifié par affinité sur les répétitions du 17 acides aminés déjà cités du LSA. Ces anticorps marquent une protéine de poids moléculaire de 200 000 Daltons.

L'hybridation du clone DG307 avec les fragments d'ADN de P. falciparum obtenus par digestion avec une enzyme de restriction, la Mung Bean nucléase, selon les conditions décrites par McCutchans (NAR, 16, 14, 6883-6896, 1988), capable de couper chaque extrémité des gènes du parasite, révèle une bande unique d'un poids moléculaire de 5 Kbases cohérent avec la taille de la protéine native mesurée dans l'extrait parasitaire.

En microscopie électronique des schizontes hépatiques obtenus par injection de sporozoites de chimpanzés, le marquage des anticorps anti-LSA purifiés par affinité, visualisés par un deuxième anticorps marqué à l'or colloïdal, révèle que la molécule LSA est distribuée : a) au stade du trophozoite hépatique et du schizonte jeune, dans des vacuoles contenant des granules qui s'ouvrent et se déversent dans la vacuole parasitophore, b) au stade du schizonte sub-mature âgés de 5 jours, en périphérie du schizonte hépatique dans les granules présents dans la vacuole parasitophore du parasite, c) chez les schizontes mûrs âgés de 6 jours à 7 jours, dans la vacuole parasitophore ainsi qu'entre les pseudo-cytomères du schizonte, puis finalement autour des mérozoites en formation.

L'étude de la réponse immunologique de sujets exposés au paludisme ainsi que d'animaux immunisés avec les protéines recombinantes et/ou synthétiques, permet de préciser la fonction biologique de la protéine LSA et de certains segments de cette protéine en particulier ceux contenus dans les peptides synthétiques.

L'immunisation de souris par les protéines LSA-R-NR et l'étude de la réponse des lymphocytes de ces souris, ainsi que l'immunisation par les peptides LSA-R de souris de différents haplotypes par des peptides LSA-R, LSA-J et LSA-NR, et enfin l'étude des réponses des lymphocytes des sujets exposés au paludisme vis-à-vis des peptides LSA-R, avaient indiqué qu'aucun épitope T pour l'homme et la souris n'est défini par la partie répétée de la molécule LSA. Des études plus détaillées montrent l'existence d'un épitope T dans le peptide LSA-R. Ainsi qu'il avait été indiqué précédemment, le peptide LSA-R constitue un excellent épitope B pour l'homme ou la souris, défini par la partie répétée de la molécule LSA, et les résultats complémentaires obtenus depuis chez plus de 500 individus exposés au paludisme indiquent que cet épitope est reconnu par les anticorps de prés de 95% des sujets étudiés, au Sénégal, en Haute Volta, à Madagascar et au Kenya.

L'étude préliminaire des lymphocytes de 5 sujets africains adultes exposés au paludisme, confirmée ensuite par l'étude détaillée de la réponse des lymphocytes périphériques de plus de 200 sujets africains adultes exposés au paludisme, ainsi que des lymphocytes de chimpanzés (Pan troglodytes) immunisés par la protéine recombinante LSA-R-NR (clone DG536), révèle qu'un épitope T de la molécule LSA est défini par la séquence d'acides aminés contenue dans le peptide synthétique LSA-NR. Deux autres épitotes T sont contenus dans les séquences des peptidides synthétiques LSA-J et LSA-R (au total 39 % de réponses positives aux épitopes T du LSA à Madagascar et 83 % au Sénégal). Des réponses prolifératives des lymphocytes humains et des lymphocytes de chimpanzé immunisé ont été observées après stimulation par ces trois peptides, a) chez le chimpanzé les lignées limphocytaires obtenues sont à 60 % du phénotype CD8+, b) chez la souris, l'injection de l'un ou l'autre de ces peptides permet de "primer" immunologiquement le système immunitaire de la souris et d'obtenir la production à taux élevé d'anticorps contre l'épitope B du peptide LSA-R après l'injection de la protéine recombinante LSA-R-NR. L'identification de ces épitopes capables de stimuler les lymphocytes T a une grande importance dans la mesure où il a été établi dans le paludisme des rongeurs que la protection induite par des sporozoites irradiés repose sur la production de lymphocytes cytotoxiques pour l'hépatocyte infecté, et capable de les détruire.

En outre, l'étude de 120 sérums de sujets africains indique aussi que le peptide LSA-NR définit un épitope B, distinct de celui que l'on trouve dans les répétitions, reconnu par environ 65 % des sujets étudiés.

Par contre le peptide LSA-TER ne constitue pas un épitope B important, il est rarement reconnu par les anticorps des sujets étudiés.

L'intérêt potentiel des épitopes T du LSA, en particulier, ceux contenus dans la partie non répétée, est en outre fortement renforcé par les résultats obtenus chez le chimpanzé:
chez le chimpanzé ayant été immunisé par trois injections à 15 jours d'intervalles d'un mélange de deux protéines reconbinantes adsorbées sur alun, d'une part la protéine recombinante LSA-R-RN (clone DG536), et d'autre part la protéine recombinante dénommée DG729S (clone DG729S faisant partie des 120 clones sus-mentionnés), plusieurs résultats importants ont pu être obtenus:
- une production d'anticorps spécifiques de chacune des deux molécules recombinantes, c'est-à-dire réagissant avec la protéine LSA-R-NR et les peptides synthétiques, ainsi qu'avec la protéine recombinante 729S, a été détectée.
- une réponse proliférative spécifique des lymphocytes a été obtenue vis-à-vis du peptide LSA-NR, et également des peptides LSA-J et LSA-TER. Les lymphocytes proliférant sont pour 60 % d'entre eux de phénotype CD8+, qui correspond en particulier à des cellules T cytotoxiques.
- Après immunisation des chimpanzés, une épreuve d'infection par injection intraveineuse de 28 millions de sporozoites de P. falciparum a été réalisée chez un chimpanzé immunisé et chez un chimpanzé témoin (ayant reçu une protéine témoin recombinante non apparentée), et des biopsies hépatiques ont été réalisées au 6ème jour après infection. L'examen des biopsies montre l'existence d'une réaction cellulaire, lympho-monocytaire, autour des schizontes hépatiques, infiltrant ces schizontes, et capables de les détruire. De telles images n'ont pas été observées chez le chimpanzé ayant reçu l'antigène contrôle.

Cette réaction cellulaire témoigne de l'existence d'épitopes T dans les molécules injectées 729S et LSA-R-NR (DG536) capables d'être exprimés dans les molécules injectées, capables d'être exprimés par les schizontes hépatiques, et d'induire un afflux cellulaire ; ce résultat est en accord avec les résultats des tests de prolifération lymphocytaires ; enfin il indique que la réponse immunitaire induite par les protéines recombinantes injectées est capable lors de la pénétration du parasite d'induire un afflux cellulaire, lui-même capable de concourir à la défense de l'organisme, en détruisant les parasites en position intra-hépatocytaire.

Une réponse proliférative spécifique des lymphocytes a été également obtenue vis-à-vis des peptides 729S-NRI et 729S-NRII.

La capacité cytolytique des lymphocytes de ce chimpanzé immunisé et protégé a été mesurée in vitro de la façon suivante : des lignées lymphocytaires ont été produites par stimulation in vitro avec les peptides LSANR et LSAJ ainsi que 729-NRI et NRII en présence d'interleukine 2. Ces lignées ont été entretenues pendant 4 semaines par restimulation en présence de cellules mononuclées autologues du même chimpanzé par les mêmes peptides et l'interleukine 2. Un test de cytolyse a été réalisé en incubant les cellules mononuclées périphériques du chimpanzé irradiées avec les mêmes peptides, puis après marquage par le chrome 51, en incubant ces cellules avec les lignées produites. Un relargage de chrome 51 traduisant la destruction des cellules cibles a été observé. Ces résultats démontrent que les épitopes T, définis ci-dessus, sont capables d'activer des lymphocytes T cytolytiques spécifiques des séquences polypeptidiques en question.

Les spécificités antigéniques et/ou immunologiques des polypeptides de l'invention sont les suivantes :
- le polypeptide 536 :
   * est reconnu :
      . par des anticorps provenant de sujets ayant le paludisme,
      . par des sérums de chimpanzés immunisés avec la protéine 536 complète recombinante,
   * co-réagit avec les polypeptides décrits dans Guérin-Marchand et al (Nature sus-mentionné) par l'intermédiaire des séquences répétitives,
   * induit la fonction d'anticorps (présence d'épitopes B)
   * induit des réponses prolifératives de lymphocytes T (présence d'épitope T) chez le chimpanzé et chez l'homme.
- les polypeptides NR et TER comprennent un épitope T important et un épitope B pour l'homme comme pour l'animal immunisé (souris et chimpanzé)
- le polypeptide LSA-J comprend un épitope B distinct de celui de la répétition de 17 acides acides aminés probablement en raison de la subsitution de S par R, et comprend un épitope T reconnu chez l'homme et le chimpanzé.

La protéine recombinante 729 S :
- est reconnue :
   * par des anticorps de sujets exposés au paludisme,
   * par des sérum de chimpanés, et de souris, immunisés par la protéine 729 S
- est mieux reconnue par les individus capables de résister à l'impadulation que par ceux qui n'y résistent pas (au nord du Sénégal, les inventeurs ont administré une cure radicale de chloroquine à 100 individus, et suivi pendant la période de transmission, de septembre à décembre, la repositivation du sang a) chez les sujets qui se sont positivés, aucun anticorps contre la protéine 729 S n'a été détecté b) chez plus de la moitié de ceux qui ne se sont pas re-positives, il existait une réponse à taux élevé contre la protéine 729S).
- est reconnu par les sérums de trois individus ayant été vaccinés par injections multiples de sporozoïtes de P. falciparum et ayant résisté à une injection d'épreuve par des sporozoïtes virulents non-irradiés, mais n'est pas reconnu par les sérums de quatre autres individus ayant été vaccinés par injections multiples de sporozoites irradiés à des doses irradiantes plus fortes et n'ayant pas résisté aux mêmes injections d'épreuve par des sporozoïtes non irradiés. Cette reconnaissance porte en particulier sur la réaction des anticorps avec le polypeptide 729S-NRII. Il existe donc une corrélation étroite entre la réponse immunitaire dirigée contre la molécule 729S et la protection induite contre le paludisme chez l'homme.

Les polypeptides 729S-NRI et 729S-NRII comportent en outre des épitoptes T importants qui sont reconnus par la majorité des sujets dont les lymphocytes ont été récemment étudiés à Madagascar et au Sénégal (18 positifs sur les 20 sujets étudiés à Madagascar et 26 sur 46 sujets étudiés au Sénégal).

Le polypeptide 729S-R contient également un épitope T reconnu par une proportion élevée d'individus d'origine sénégalaise et malgache (30 à 60 % des individus étudiés), mais en outre, il définit un épitope B majeur de la molécule puisque les anticorps de 96 à 100 % des sujets étudiés au Sénégal, au Cameroun et à Madagascar reconnaissent ce peptide et que le taux d'anticorps observé est extrêmement élevé.

Il apparaîtra immédiatement à l'homme de métier que dans les séquences nucléiques sus-mentionnées, certains des nucléotides peuvent être remplacés par d'autres en raison de la dégénéréscence du code génétique sans que pour autant les peptides codés ne soient modifiés. Toutes ces séquences nucléotidiques, ainsi que celles qui codent pour des polypeptides qui différent des précédents par un ou plusieurs acides aminés sans que leur activité immunogénique propre ne soit modifiée de façon semblable, font partie de l'invention. Il en va naturellement de même des séquences nucléotidiques qui peuvent être reconstituées et qui sont capables de coder pour des oligoméres tels qu'ils ont été définis ci-après. Les motifs monomères sont liés directement bout à bout ou par l'intermédiaire de séquences peptidiques sans effet sur les propriétés immunogéniques des oligoméres ainsi formés.

Des bactéries hébergeant les susdits clones DG 199 et DG 307 ont été déposées à la Collection Nationale des Cultures de Microorganismes de l'Institut Pasteur de Paris (CNCM), le 22 juillet 1986 respectivement sous les numéros I-580 et I-581. Des bactéries hébergeant le clone DG 145 ont été déposées le 15 septembre 1986 sous le numéro I-606. Les clones DG 536 et DG 729 ont été respectivement déposés le 17 janvier 1991 sous le numéro I-1027 et le 17 janvier 1991 sous le numéro I-1028.

Dans la formule qui précède, il est fait application de la nomenclature internationale désignant chacun des aminoacides naturels par une lettre unique, notamment selon le tableau des correspondances qui suit :
- M: Méthionine
- L: Leucine
- I: Isoleucine
- V: Valine
- F: Phenylalanine
- S: Sérine
- P: Proline
- T: Théorine
- A: Alanine
- Y: Tyrosine
- H: Histidine
- Q: Glutamine
- N: Asparagine
- K: Lysine
- D: Acide Aspartique
- E: Acide glutamique
- C: Cystéine
- W: Tryptophane
- R: Arginine
- G: Glycine

## Revendications

1. Molécule, ou polypeptide, comportant au moins une séquence peptidique qui comprend au moins 4 à 5 acides aminés et qui est porteuse de tout ou partie d'un ou plusieurs épitopes caractéristiques d'une protéine produite dans les hépatocytes infectés par P. falciparum, caractérisée en ce que cette séquence peptidique comprend :
a) soit tout ou partie de l'enchaînement des 153 premiers acides aminés représentés sur la figure 7, et correspondant à la partie 5' du gène LSA ;
b) soit tout ou partie de l'enchaînement d'acides aminés suivant : 
RDELFNELLNSVDVNGEVKENILEESQVNDDIFNSLVKSVQQEQQHNVEEKVE
ESVEENDEESVEENVEENVEENDDGSVASSVEESIASSVDESIDSSIEENVAP
TVEEIVAPTVEEIVAPSVVEKCAPSVEESVAPSVEESVAEMLKER
 représenté sur la figure 3 et désigné ci-après par le polypeptide 729S ;
c) soit tout ou partie des 279 derniers acides aminés représentés sur la figure 10 et correspondant à la partie 3' du gène LSA ;
d) soit tout ou partie de l'enchaînement d'acides aminés suivant : 
RKADTKKNLERKKEHGDILAEDLYGRLEIP
AIELPSENERGYYIPHQSSLPQDNRGNSRD
SKEISIIEKTNRESITTNVEGRRDIHKGHL
EEKKDGSIKPEQKEDKS
 représenté sur la figure 10.

2. Molécule selon la revendication 1, caractérisée en ce que ladite séquence peptidique est porteuse de tout ou partie d'un ou plusieurs épitope(s) T des protéines produites au stade hépatique de P. falciparum.

3. Molécule selon la revendication 1 ou 2, caractérisée en ce que la séquence peptidique a) de la revendication 1 est suivie par tout ou partie d'un ou plusieurs des enchaînements de 17 acides aminés de formule : 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 dans laquelle :
. X₁ est "Ser" ou "Arg"
. X₂ est "Glu" ou "Asp"
. X₃ est "Arg" ou "Leu"
. X₄ est "Glu" ou "Gly"

4. Molécule selon la revendication 1 ou 2, caractérisée en ce que la séquence peptidique c) ou la séquence peptidique d) de la revendication 1 est précédée par tout ou partie d'un ou plusieurs des enchaînements de 17 acides aminés de formule : 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 dans laquelle :
. X₁ est "Ser" ou "Arg"
. X₂ est "Glu" ou "Asp"
. X₃ est "Arg" ou "Leu"
. X₄ est "Glu" ou "Gly"

5. Molécule selon la revendication 4 caractérisée par tout ou partie de l'enchaînement d'acides aminés suivant :

LQEQQRDLEQRKADTKKNLERKKEHGDILAEDLYGRLEIP
AIELPSENERGYYIPHQSSLPQDNRGNSRDSKEISIIEKT
NRESITTNVEGRRDIHKGHLEEKKDGSIKPEQKEDKS

6. Molécule selon la revendication 5, caractérisée par tout ou partie de l'enchaînement d'acides aminés suivant :

DTKKNLERKKEHGDILAEDLYGRLEIP

7. Molécule selon la revendication 4 caractérisée par tout ou partie de l'enchaînement d'acides aminés suivant :

ERRAKEKLQEQQRDLEQRKADTKK

8. Molécule selon la revendication 1, caractérisée par tout ou partie de l'enchaînement d'acides aminés suivant : NSRDSKEISIIEKTNRESITTNVEGRRDIHK de la séquence c) de la revendication 1.

9. Molécule selon la revendication 1 ou 2, caractérisée par tout ou partie de l'enchaînement d'acides aminés suivant : RDELFNELLNSVDVNGEVKENILEESQVNDDIFNSLVKSVQQEQQHN de la séquence b) de la revendication 1.

10. Molécule selon la revendication 1 ou 2, caractérisée en ce qu'elle répond à tout ou partie de l'un des enchaînements d'acides aminés suivants : 
DELFNELLNSVDVNGEVKENILEESQ,
LEESQVNDDIFSNSLVKSVQQEQQHNV,
VEKCAPSVEESVAPSVEESVAEMLKER,
 de la séquence b) de la revendication 1.

11. Molécule selon la revendication 1 ou 2, caractérisée en ce que cette séquence peptidique est représentée par tout ou partie de l'enchaînement des 153 premiers acides aminés représentés sur la figure 7, et tout ou partie des 279 derniers acides aminés représentés sur la figure 10 et correspondant à la partie 3' du gène LSA.

12. Molécule selon la revendication 1 ou 2, caractérisée en ce que cette séquence peptidique comprend successivement :
- tout ou partie de l'enchaînement des 153 premiers acides aminés représentés sur la figure 7, et correspondant à la partie 5' du gène LSA,
- tout ou partie d'un ou plusieurs des enchaînements de 17 acides aminés de formule : 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 dans laquelle :
. X₁ est "Ser" ou "Arg"
. X₂ est "Glu" ou "Asp"
. X₃ est "Arg" ou "Leu"
. X₄ est "Glu" ou "Gly"
- et tout ou partie des 279 derniers acides aminés représentés sur la figure 10 et correspondant à la partie 3' du gène LSA.

13. Séquence peptidique dérivée d'une molécule selon l'une des revendications 1 à 12, cette séquence présentant des modifications par substitution de 40 % au maximum des acides aminés tout en conservant l'activité biologique de la molécule sus-mentionnée, notamment l'induction d'une réponse des lymphocytes T, en particulier des lymphocytes T cytotoxiques.

14. Composition immunogène caractérisée par l'association d'une molécule conforme à l'une quelconque des revendications 1 à 13, en association avec un véhicule pharmaceutiquement acceptable.

15. Composition de vaccin dirigée contre le paludisme, contenant entre autres principes immunogènes, une molécule conforme à l'une quelconque des revendications 1 à 13.

16. Séquence de nucléotides correspondant selon le code génétique universel à une séquence peptidique telle que définie dans l'une des revendications 1 à 13.

17. Anticorps, polyclonaux ou monoclonaux, reconnaissant spécifiquement une molécule selon l'une quelconque des revendications 1 à 13.

18. Procédé de détection in vitro du paludisme chez un individu susceptible d'être infecté par P. falciparum qui comprend la mise en contact d'un tissu ou d'un fluide biologique prélevé chez un individu avec une molécule selon l'une des revendications 1 à 13 dans des conditions permettant une réaction immunologique in vitro entre ladite molécule et les anticorps éventuellement présents dans le tissu biologique, et la détection in vitro des complexes antigènes-anticorps éventuellement formés.

19. Procédé de détection in vitro du paludisme chez un individu susceptible d'être infecté par P. falciparum qui comprend la mise en contact d'un tissu ou d'un fluide biologique prélevé chez un individu avec des anticorps selon la revendication 17 dans des conditions permettant une réaction immunologique in vitro entre lesdits anticorps et les protéines spécifiques de P. falciparum éventuellement présentes dans le tissu biologique, et la détection in vitro des complexes antigènes-anticorps éventuellement formés.

20. Coffret ou kit pour le diagnostic in vitro du paludisme, caractérisé en ce qu'il comprend :
- une ou plusieurs molécule(s) selon l'une des revendications 1 à 13,
- les réactifs pour la constitution du milieu propice à la réalisation de la réaction immunologique,
- les réactifs permettant la détection des complexes antigènes-anticorps produits par la réaction immunologique.

21. Coffret ou kit pour le diagnostic in vitro du paludisme, caractérisé en ce qu'il comprend :
- des anticorps selon la revendication 17,
- les réactifs pour la constitution du milieu propice à la réalisation de la réaction immunologique,
- les réactifs permettant la détection des complexes antigènes-anticorps produits par la réaction immunologique.

22. Coffret ou kit pour le diagnostic selon la revendication 20 ou 21, caractérisé en ce que :
- les réactifs permettant la détection des complexes antigènes-anticorps portent un marqueur ou sont susceptibles d'être reconnus à leur tour par un réactif marqué dans le cas où les anticorps sus-mentionnés ne sont pas marqués.

23. Vecteur recombinant pour le clonage d'une séquence nucléotidique selon la revendication 16, et/ou l'expression du polypeptide codé par la susdite séquence contenant au moins une séquence de nucléotides selon la revendication 16, dans l'un des sites non essentiel pour sa réplication, ledit vecteur étant de type plasmide, cosmide ou phage.

24. Vecteur selon la revendication 23, caractérisé en ce qu'il s'agit du plasmide DG536 déposé à la CNCM sous le numéro I-1027 le 17 janvier 1991, ainsi que du plasmide DG729S déposé à la CNCM sous le numéro I-1028 le 17 janvier 1991.

25. Composition pharmaceutique comportant à titre de substance active un ou plusieurs anticorps polyclonaux ou monoclonaux selon la revendication 17 en association avec un véhicule pharmaceutiquement acceptable.

26. Utilisation d'une molécule selon l'une quelconque des revendications 1 à 13 pour la préparation d'un vaccin destiné à la prévention du paludisme.

27. Utilisation d'un ou plusieurs anticorps polyclonaux ou monoclonaux selon la revendication 17 pour la préparation d'un médicament destiné au traitement du paludisme.

## Patentansprüche

1. Molekül oder Polypeptid, das mindestens eine Peptidsequenz aufweist, die mindestens 4 bis 5 Aminosäuren umfaßt und die Trägerin eines ganzen oder eines Teiles von einem oder mehreren Epitopen ist, die charakteristisch für ein Protein sind, das in mit P. falciparum infizierten Hepatocyten erzeugt wird, dadurch gekennzeichnet, daß diese Peptidsequenz umfaßt:
a) entweder die ganze oder Teile der Kette der ersten 153 Aminosäuren, die in Figur 7 dargestellt sind und dem 5'-Teil des LSA-Gens entsprechen;
b) entweder die ganze oder Teile der folgenden Aminosäurekette: 
RDELFNELLNSVDVNGEVKENILEESQVNDDIFNSLVKSVQQEQQHNVEEKVE
ESVEENDEESVEENVEENVEENDDGSVASSVEESIASSVDESIDSSIEENVAP
TVEEIVAPTVEEIVAPSVVEKCAPSVEESVAPSVEESVAEMLKER
 die in Figur 3 dargestellt ist und im folgenden als Polypeptid 729S bezeichnet ist;
c) entweder alle oder Teile der letzten 279 Aminosäuren, die in Figur 10 dargestellt sind und die dem 3'-Teil des LSA-Gens entsprechen;
d) entweder die ganze oder Teile der folgenden Aminosäurekette: 
RKADTKKNLERKKEHGDILAEDLYGRLEIP
AIELPSENERGYYIPHQSSLPQDNRGNSRD
SKEISIIEKTNRESITTNVEGRRDIHKGHL
EEKKDGSIKPEQKEDKS
 dargestellt in der Figur 10.

2. Molekül nach Anspruch 1, dadurch gekennzeichnet, daß die besagte Peptidsequenz Trägerin des ganzen oder eines Teils von einem oder mehreren Epitopen T der Proteine ist, die während des hepatischen Stadiums von P. falciparum hergestellt werden.

3. Molekül nach Anspruch 1 oder 2, dadurch gekennzeichnet, daß der Peptidsequenz a) des Anspruchs 1 der ganze oder ein Teil von einer oder mehreren Ketten aus 17 Aminosäuren der Formel folgt: 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 wobei:
X₁ "Ser" oder "Arg" ist,
X₂ "Glu" oder "Asp" ist,
X₃ "Arg" oder "Leu" ist,
X₄ "Glu" oder "Gly" ist.

4. Molekül nach Anspruch 1 oder 2, dadurch gekennzeichnet, daß der Peptidsequenz c) oder der Peptidsequenz d) des Anspruchs 1 ganz oder teilweise eine oder mehrere Ketten von 17 Aminosäuren der Formel vorausgeht: 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 wobei:
X₁ "Ser" oder "Arg" ist,
X₂ "Glu" oder "Asp" ist,
X₃ "Arg" oder "Leu" ist,
X₄ "Glu" oder "Gly" ist.

5. Molekül nach Anspruch 4, gekennzeichnet durch die ganze oder einen Teil der folgenden Aminosäurekette:

LQEQQRDLEQRKADTKKNLERKKEHGDILAEDLYGRLEIP
AIELPSENERGYYIPHQSSLPQDNRGNSRDSKEISIIEKT
NRESITTNVEGRRDIHKGHLEEKKDGSIKPEQKEDKS

6. Molekül nach Anspruch 5, gekennzeichnet durch die ganze oder einen Teil der folgenden Aminosäurekette:

DTKKNLERKKEHGDILAEDLYGRLEIP

7. Molekül nach Anspruch 4, gekennzeichnet durch die ganze oder einen Teil der folgenden Aminosäurekette:

ERRAKEKLQEQQRDLEQRKADTKK

8. Molekül nach Anspruch 1, gekennzeichnet durch die ganze oder einen Teil der folgenden Aminosäurekette: NSRDSKEISIIEKTNRESITTNVEGRRDIHK der Sequenz c) vom Anspruch 1.

9. Molekül nach Anspruch 1 oder 2, gekennzeichnet durch die ganze oder einen Teil der folgenden Aminosäurekette: RDELFNELLNSVDVNGEVKENILEESQVNDDIFNSLVKSVQQEQQHN der Sequenz b) nach Anspruch 1.

10. Molekül nach Anspruch 1 oder 2, dadurch gekennzeichnet, daß es ganz oder teilweise einer der folgenden Aminosäureketten entspricht: 
DELFNELLNSVDVNGEVKENILEESQ,
LEESQVNDDIFSNSLVKSVQQEQQHNV,
VEKCAPSVEESVAPSVEESVAEMLKER,
 der Sequenz b) nach Anspruch 1.

11. Molekül nach Anspruch 1 oder 2, dadurch gekennzeichnet, daß diese Peptidsequenz dargestellt ist durch die ganze oder einen Teil der Kette der ersten 153 Aminosäuren, dargestellt in Figur 7, und allen oder einem Teil der letzten 279 Aminosäuren, die in Figur 10 dargestellt sind und dem 3'-Teil des LSA-Gens entsprechen.

12. Molekül nach Anspruch 1 oder 2, dadurch gekennzeichnet, daß diese Peptidsequenz hintereineinander umfaßt:
- die ganze oder einen Teil der Kette der ersten 153 Aminosäuren, die in Figur 7 dargestellt sind und dem 5'-Teil des LSA-Gens entsprechen,
- alle oder einen Teil einer oder mehrerer Ketten von 17 Aminosäuren mit der Formel 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
 
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 wobei
X₁ "Ser" oder "Arg" ist,
X₂ "Glu" oder "Asp" ist,
X₃ "Arg" oder "Leu" ist,
X₄ "Glu" oder "Gly" ist,
- und alle oder einen Teil der letzten 279 Aminosäuren, die in Figur 10 dargestellt sind und dem 3'-Teil des LSA-Gens entsprechen.

13. Peptidsequenz, abgeleitet von einem Molekül nach einem der Ansprüche 1 bis 12, wobei die Sequenz Änderungen durch Substitution von maximal 40 % der Aminosäuren zeigt, wobei die biologische Aktivität des oben genannten Moleküls erhalten bleibt, vor allem die Induktion einer T-Lymphocytenantwort, besonders von cytotoxischen T-Lymphocyten.

14. Immunogene Zusammensetzung, gekennzeichnet durch die Kombination eines Moleküls gemäß einem der Ansprüche 1 bis 13 mit einem pharmazeutisch verträglichen Träger.

15. Impfstoff gegen Malaria, umfassend, neben anderen immunogenen Bestandteilen, ein Molekül gemäß einem der Ansprüche 1 bis 13.

16. Nucleotidsequenz, die nach dem universellen genetischen Code einer Peptidsequenz entspricht, die in einem der Ansprüche 1 bis 13 definiert ist.

17. Polyclonale oder monoclonale Antikörper, die spezifisch ein Molekül nach einem der Ansprüche 1 bis 13 erkennen.

18. In vitro-Nachweisverfahren von Malaria in einem Individuum, das wahrscheinlich mit P. falciparum infiziert ist, umfassend das Inkontaktbringen eines Gewebes oder einer biologischen Flüssigkeit, entnommen von einem Individuum, mit einem Molekül nach einem der Ansprüche 1 bis 13 unter Bedingungen, die eine immunologische in vitro-Reaktion zwischen dem Molekül und den Antikörpern, die vielleicht im biologischen Gewebe vorhanden sind, erlauben, und der in vitro-Nachweis der möglicherweise gebildeten Antigen-Antikörper-Komplexe.

19. In vitro-Nachweisverfahren von Malaria in einem Individuum, das wahrscheinlich mit P. falciparum infiziert ist, umfassend das Inkontaktbringen eines Gewebes oder einer biologischen Flüssigkeit, entnommen von einem Individuum, mit Antikörpern nach Anspruch 17 unter Bedingungen, die eine immunologische in vitro-Reaktion zwischen den Antikörpern und möglicherweise im biologischen Gewebe vorhandenen spezifischen Proteinen des P. falciparum erlauben, und der in vitro-Nachweis der möglicherweise gebildeten Antigen-Antikörper-Komplexe.

20. Kit zur in vitro-Diagnose von Malaria, dadurch gekennzeichnet, daß es umfaßt:
- ein oder mehrere Moleküle nach einem der Ansprüche 1 bis 13,
- Reagenzien zur Herstellung eines günstigen Mediums zur Durchführung der immunologischen Reaktion,
- Reagenzien, die den Nachweis der Antigen-Antikörper-Komplexe, die durch die immunologische Reaktion erzeugt wurden, erlauben.

21. Kit zur in vitro-Diagnose von Malaria, dadurch gekennzeichnet, daß es umfaßt:
- Antikörper nach Anspruch 17,
- Reagenzien zur Herstellung eines günstigen Mediums zur Durchführung der immunologischen Reaktion,
- Reagenzien, die den Nachweis der Antigen-Antikörper-Komplexe, die durch die immunologische Reaktion erzeugt wurden, erlauben.

22. Diagnostisches Kit nach Anspruch 20 oder 21, gekennzeichnet durch:
- Reagenzien, die den Nachweis von Antigen-Antikörper-Komplexen erlauben, die einen Marker tragen oder die ihrerseits fähig sind von einem markierten Reagenz erkannt zu werden, falls die oben genannten Antikörper nicht markiert sind.

23. Rekombinanter Vektor zur Clonierung einer Nucleotidsequenz nach Anspruch 16 und/oder zur Expression eines Polypeptids, das durch die obengenannte Sequenz codiert ist, der an einer für die Replikation nicht essentiellen Stelle mindestens eine Nucleotidsequenz nach Anspruch 16 umfaßt, wobei der Vektor vom Plasmid-, Cosmid- oder Phagentyp ist.

24. Vektor nach Anspruch 23, dadurch gekennzeichnet, daß es sich sowohl um das Plasmid DG536, das am 17. Januar 1991 beim CNCM unter der Nummer I-1027 hinterlegt wurde, als auch um das Plasmid DG729S handelt, das am 17. Januar 1991 beim CNCM unter der Nummer I-1028 hinterlegt wurde.

25. Arzneimittel, das als Wirkstoff eine oder mehrere polyclonale oder monoclonale Antikörper nach Anspruch 17 in Verbindung mit einem pharmazeutisch verträglichen Träger umfaßt.

26. Verwendung eines Moleküls nach einem der Ansprüche 1 bis 13, zur Herstellung eines Impfstoffs zur Vorbeugung gegen Malaria.

27. Verwendung von einem oder mehreren polyclonalen oder monoclonalen Antikörpern nach Anspruch 17 zur Herstellung eines Medikaments zur Behandlung von Malaria.

## Claims

1. A molecule or a polypeptide comprising at least one peptide sequence containing at least 4 or 5 amino acids and which carries all or part of one or more epitopes which are characteristic of a protein produced in hepatocytes infected with *P. falciparum,* characterized in that said peptide sequence comprises:
a) either all or part of the concatenation of the first 153 amino acids shown in Figure 7 and corresponding to the 5' portion of the LSA gene;
b) or all or part of the following amino acid concatenation: 
RDELFNELLNSVDVNGEVKENILEESQVNDDIFNSLVKSVQQEQQH
NVEEKVEESVEENDEESVEENVEENVEENDDGSVASSVEESIASSV
DESIDSSIEENVAPTVEEIVAPTVEEIVAPSVVEKCAPSVEESVAPSV
EESVAEMLKER
 shown in Figure 3 and hereinafter termed polypeptide 729S;
c) or all or part of the last 279 amino acids shown in Figure 10 and corresponding to the 3' portion of the LSA gene;
d) or all or part of the following amino acid concatenation: 
RKADTKKNLERKKEHGDILAEDLYGRLEIPAIELPSENERGYYIPHQ
SSLPQDNRGNSRDSKEISIIEKTNRESITTNVEGRRDIHKGHLEEKKD
GSIKPEQKEDKS
 shown in Figure 10.

2. A molecule according to claim 1, characterized in that said peptide sequence carries all or part of one or more T epitope(s) of proteins produced in the liver stage of *P. falciparum*.

3. A molecule according to claim 1 or claim 2, characterized in that the peptide sequence a) of claim 1 is followed by all or part of one or more concatenations of 17 amino acids with formula: 
X1DLEQX2RX3AKEKLQX4QQ
QX1DLEQX2RX3AKEKLQX4Q
QQX1DLEQX2RX3AKEKLQX4
 
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 where:
• X₁ is "Ser" or "Arg";
• X₂ is "Glu" or "Asp";
• X₃ is "Arg" or "Leu";
• X₄ is "Glu" or "Gly".

4. A molecule according to claim 1 or claim 2, characterized in that the peptide sequence c) or the peptide sequence d) of claim 1 is preceded by all or part of one or more concatenations of 17 amino acids with formula: 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
 
KEKLQX4QQX1DLEQX2RX3A
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 where:
• X₁ is "Ser" or "Arg";
• X₂ is "Glu" or "Asp";
• X₃ is "Arg" or "Leu";
• X₄ is "Glu" or "Gly".

5. A molecule according to claim 4, characterized by all or part of the following concatenation of amino acids:

LQEQQRDLEQRKADTKKNLERKKEHGDILAEDLYGRLEIPAIELPSE
NERGYYIPHQSSLPQDNRGNSRDSKEISIIEKTNRESITTNVEGRRDI
HKGHLEEKKDGSIKPEQKEDKS

6. A molecule according to claim 5, characterized by all or part of the following concatenation of amino acids:

DTKKNLERKKEHGDILAEDLYGRLEIP

7. A molecule according to claim 4, characterized by all or part of the following concatenation of amino acids:

ERRAKEKLQEQQRDLEQRKADTKK

8. A molecule according to claim 1, characterized by all or part of the following concatenation of amino acids: NSRDSKEISIIEKTNRESITTNVEGRRDIHK from sequence c) of claim 1.

9. A molecule according to claim 1 or claim 2, characterized by all or part of the following concatenation of amino acids: RDELFNELLNSVDVNGEVKENILEESQVNDDIFNSLVKSVQQEQQHN from sequence b) of claim 1.

10. A molecule according to claim 1 or claim 2, characterized in that all or part of it is constituted by one of the following concatenations of amino acids: 
DELFNELLNSVDVNGEVKENILEESQ;
LEESQVNDDIFSNSLVKSVQQEQQHNV;
VEKCAPSVEESVAPSVEESVAEMLKER
 from sequence b) of claim 1.

11. A molecule according to claim 1 or claim 2, characterized in that said peptide sequence is represented by all or part of the concatenation of the first 153 amino acids shown in Figure 7, and all or part of the last 279 amino acids shown in Figure 10 and corresponding to the 3' portion of the LSA gene.

12. A molecule according to claim 1 or claim 2, characterized in that said peptide sequence comprises, in succession:
• all or part of the concatenation of the first 153 amino acids shown in Figure 7, and corresponding to portion 5' of the LSA gene;
• all or part of one or more concatenations of 17 amino acids with formula: 
X1DLEQX2RX3AKEKLQX4QQ
QX1DLEQX2RX3AKEKLQX4Q
QQX1DLEQX2RX3AKEKLQX4
X4QQX1DLEQX2RX3AKEKLQ
QX4QQX1DLEQX2RX3AKEKL
LQX4QQX1DLEQX2RX3AKEK
KLQX4QQX1DLEQX2RX3AKE
EKLQX4QQX1DLEQX2RX3AK
KEKLQX4QQX1DLEQX2RX3A
 
AKEKLQX4QQX1DLEQX2RX3
X3AKEKLQX4QQX1DLEQX2R
RX3AKEKLQX4QQX1DLEQX2
X2RX3AKEKLQX4QQX1DLEQ
QX2RX3AKEKLQX4QQX1DLE
EQX2RX3AKEKLQX4QQX1DL
LEQX2RX3AKEKLQX4QQX1D
DLEQX2RX3AKEKLQX4QQX1
 where:
• X₁ is "Ser" or "Arg";
• X₂ is "Glu" or "Asp";
• X₃ is "Arg" or "Leu";
• X₄ is "Glu" or "Gly";
• and all or part of the last 279 amino acids shown in Figure 10 and corresponding to the 3' portion of the LSA gene.

13. A peptide sequence derived from a molecule according to any one of claims 1 to 12, said sequence being modified by substituting a maximum of 40% of the amino acids while preserving the biological activity of said molecule, in particular that of inducing a T lymphocyte response, more particularly in cytotoxic T lymphocytes.

14. An immunogenic composition characterized by a combination of a molecule according to any one of claims 1 to 13 with a pharmaceutically acceptable vehicle.

15. A vaccine composition directed against malaria, containing a molecule according to any one of claims 1 to 13, among other immunogenic constituents.

16. A nucleotide sequence which, using the universal genetic code, corresponds to a peptide sequence as defined in any one of claims 1 to 13.

17. Polyclonal or monoclonal antibodies specifically recognising a molecule according to any one of claims 1 to 13.

18. A method for *in vitro* detection of malaria in an individual susceptible of being infected with *P. falciparum,* which comprises bringing a biological tissue or fluid taken from an individual into contact with a molecule according to any one of claims 1 to 13 under conditions enabling an *in vitro* immunological reaction to occur between said molecule and any antibodies which may be present in the biological tissue, and *in vitro* detection of antigen-antibody complexes which may be formed.

19. A method for *in vitro* detection of malaria in an individual susceptible of being infected with *P. falciparum,* which comprises bringing a biological tissue or fluid taken from an individual into contact with antibodies according to claim 17 under conditions enabling an *in vitro* immunological reaction to occur between said antibodies and any specific *P. falciparum* proteins which may be present in the biological tissue, and *in vitro* detection of antigen-antibody complexes which may be formed.

20. A kit for diagnosing malaria *in vitro,* characterized in that it comprises:
• one or more molecule(s) according to any one of claims 1 to 13;
• reactants for constituting a suitable medium for carrying out an immunological reaction;
• reactants enabling antigen-antibody complexes produced by the immunological reaction to be detected.

21. A kit for diagnosing malaria *in vitro,* characterized in that it comprises:
• antibodies according to claim 17;
• reactants for constituting a suitable medium for carrying out an immunological reaction;
• reactants enabling antigen-antibody complexes produced by the immunological reaction to be detected.

22. A diagnosis kit according to claim 20 or claim 21, characterized in that:
• the reactants enabling antigen-antibody complexes to be detected carry a label or can in turn be recognised by a labelled reactant when said antibodies are not labelled.

23. A recombinant vector for cloning a nucleotide sequence according to claim 16, and/or for expressing the polypeptide coded by said sequence, containing at least one nucleotide sequence according to claim 16, in a site which is not essential for replication, said vector being a plasmid, a cosmid or a phage.

24. A vector according to claim 23, characterized in that it is plasmid DG536 deposited at the CNCM on 17^{th} January 1991 with accession number 1-1027, also plasmid DG729S deposited at the CNCM on I7^{th} January 1991 with accession number I-1028.

25. A pharmaceutical composition comprising one or more polyclonal or monoclonal antibodies according to claim 17 as an active ingredient, combined with a pharmaceutically acceptable vehicle.

26. Use of a molecule according to any one of claims 1 to 13 to prepare a vaccine for the prevention of malaria.

27. Use of one or more polyclonal or monoclonal antibodies according to claim 17 for the preparation of a drug intended for the treatment of malaria.
